(19) Europäisches Patentamt
European Patent Office
Office européen des brevets

(11) **EP 4 070 813 A1**

(12) **EUROPEAN PATENT APPLICATION**
published in accordance with Art. 153(4) EPC

(43) Date of publication:
**12.10.2022 Bulletin 2022/41**

(21) Application number: **20859623.9**

(22) Date of filing: **07.12.2020**

(51) International Patent Classification (IPC):
***A61K 39/12*** (2006.01)      ***C07K 14/025*** (2006.01)

(52) Cooperative Patent Classification (CPC):
**C07K 14/005;** A61K 2039/53; A61K 2039/585;
C07K 2319/00; C12N 2710/20022;
C12N 2710/20023; C12N 2710/20034

(86) International application number:
**PCT/BR2020/050516**

(87) International publication number:
**WO 2021/108884 (10.06.2021 Gazette 2021/23)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **05.12.2019 BR 102019025802**

(71) Applicant: **INSTITUTO BUTANTAN**
**05503900 São Paulo - SP (BR)**

(72) Inventors:
• **CIANCIARULLO, Aurora Marques**
**05351-015 São Francisco - SP (BR)**
• **SASAKI, Érica Akemi Kavati**
**04726-160 Vila Cruzeiro - SP (BR)**

(74) Representative: **De Clercq & Partners**
**Edgard Gevaertdreef 10a**
**9830 Sint-Martens-Latem (BE)**

(54) **PROCESS FOR PRODUCING A PROPHYLACTIC AND THERAPEUTIC DNA IMMUNOLOGICAL COMPOSITION FOR HPV AND CANCERS ASSOCIATED WITH THE VIRUS, HYBRID PROTEIN, EXPRESSION VECTOR, IMMUNOLOGICAL COMPOSITION AND USES THEREOF**

(57)     The present invention relates to the prophylactic and therapeutic vaccine against HPV and cancers associated with the virus, aimed at people seeking prevention or those already infected with HPV who have developed cancer. It also refers to DNA expression vectors that can efficiently produce the L2 protein of the HPV16 virus capsid, in addition to the viral E6 oncoprotein associated with human papillomavirus tumors. In particular, this invention relates to obtaining recombinant fusion or hybrid proteins, through gene cloning into expression vectors, produced by the genetic translation of fused genes, formed by the combination of nucleic acid regulatory sequences of one or more genes, with the protein coding sequences of one or more genes, used to generate two distinct types of responses: lasting humoral immune response, capable of stimulating the production of specific anti-L2 and anti-E6 antibodies against HPV (prophylactic action), as well as activate the cellular immune response, to fight tumor cells and induce the expression of TNF cytokines (Tumor Necrosis Factor) or Tumor Necrosis Factor (therapeutic action).

Figure 1

**Description**

FIELD OF THE INVENTION

The present invention relates to the prophylactic and therapeutic vaccine against HPV and cancers associated with the virus, intended for anyone interested in preventing the disease, as well as for people infected by HPV who developed cancer. The invention also refers to DNA expression vectors that can efficiently produce the L2 protein of the HPV16 virus capsid, in addition to the viral oncoprotein E6 associated with the development of tumors caused by human *Papillomavirus.* In particular, this invention relates to obtaining recombinant fusion or hybrid proteins, through gene cloning into expression vectors, produced by the genetic translation of fused genes, formed by the combination of nucleic acid regulatory sequences of one or more genes, with the protein coding sequences of one or more genes, used to generate two distinct types of responses: lasting humoral immune response, capable of stimulating the production of specific anti-L2 and anti-E6 antibodies against HPV (prophylactic action), as well as activating the cellular immune response, to fight already established tumor cells and induce the expression of TNF cytokines (Tumor Necrosis Factor) or Tumor Necrosis Factor (therapeutic action).

BACKGROUND OF THE INVENTION

Cervical cancer is the third most common cancer in women worldwide, it being the second leading cause of death in women from cancer. The two most prevalent viral types in the population are HPV16 and HPV18, detected in the most invasive cancers. They are mainly caused by persistent high-risk HPV (human *Papillomavirus*) infection (GUAN P, HOWELL-JONESR, L] N, et al. Human Papillomavirus types in 115,789 HPV-positive women: a meta-analysis from cervical infection to cancer. Int J. Cancer, v. 131, p. 2349-2359, 2012) .

Despite the availability of prophylactic vaccines, HPV infections remain extremely common worldwide. It is estimated that cervical cancer currently affects more than 1.4 million women worldwide, leading to the death of more than 300,000 women a year, mainly affecting developing countries (WHO. Strategic Advisory Group of Experts (SAGE) on immunization WHO. World Health Organization, 2017; DE SANJOSÉ S, DIAZ M, CASTELLSAGUÉ, et al. Worldwide prevalence and genotype distribution of cervical human Papillomavirus DNA in women with normal cytology: a meta-analysis. Lancet Infect Dis, v. 7, p 453-459, 2007).

Among the factors responsible for this high mortality, the high cost and requirement for refrigeration of vaccines are the ones that most restrict the implementation of large-scale preventive immunization in developing countries, where approximately 87% of deaths from cervical cancer occur (GLOBOCANE, Estimated cancer incidence, mortality and prevalence worldwide in 2012, v 1.0 [internet, 2012). The price of the bivalent HPV vaccine is approximately R$ 200 per dose and that of the tetravalent vaccine is approximately R$ 300 per dose in private clinics, requiring 2 to 3 doses with booster every 5 to 8 years (Cianciarullo AM. Prophylaxis against human Papillomavirus. Rev. Sodebras, v. 9, n. 100, p. 8-15, 2014. Available at: http://www.sodebras.com.br/edicoes/N100.pdf) .

Nowadays, the main form for preventing cervical cancer is the performance of periodic cytological examinations, Pap smears and the prophylactic vaccination of pre-adolescent girls and boys. In Brazil, the prophylactic Gardasil® quadrivalent vaccine was recently made available by the Ministry of Health, free of charge through SUS (Unified Health System) for boys and girls from 9 to 14 years old, as well as for men and women from 9 to 26 years old living with HIV or AIDS, patients who received organ and bone marrow transplants and people undergoing cancer treatment (Cianciarullo AM. Prophylaxis against human Papillomavirus. Rev. Sodebras, v. 9, n. 100, p. 8-15, 2014 Available at: http://www.sodebras.com.br/edicoes/N100.pdf; INCA. 2017. Available at: http://portalsaude.saude.gov.br.

The vaccine can also be used by older people, however, said vaccine is only available in private vaccination clinics. It is indicated for girls and women between 9 and 45 years of age, if it is the quadrivalent vaccine, or any age above 9 years, if it is the bivalent vaccine; boys and men between 9 and 26 years of age, with the quadrivalent vaccine. In addition, the vaccine can be used by people undergoing treatment or who have had HPV infection, as it can protect against other types of HPV viruses, and prevent the formation of new genital warts and the risk of cancer (see Cianciarullo AM. human Papillomavirus.Sodebras, v. 9, n. 100, p. 8-15, 2014. Available at: http://www.sodebras.com.br/edicoes/N100.pdf; INCA. 2017. Available at:http://portalsaude.saude.gov.br.

However, such actions aim to prevent HPV infection, as there is no specific treatment already established against infections and injuries. Among the proteins expressed by HPV, we highlight the L2 and E6 that are the targets of this work. The L2 protein is present in the viral capsid and is well conserved among different types of HPV, while E6 is an oncogenic protein capable of inducing the malignant transformation of infected cells. The complete viral genome is required for the malignancy to establish itself (Herrero R., Gonzalez P, Markowitz LE. Present status of human Papillomavirus vaccine development and implementation. Lancet Oncol, v. 16, p. E206-216, 2015; Wang D, Li Z, Xiao J, et al. Identification of broad-genotype HPV L2 neutralization site for Pan-HPV vaccine development by a cross-neutralizing antibody. PLOS One, v. 10, p. E0123944, 2015; Vande Pol SB, Klingelhutza J Papillomavirus E6 oncoproteins Virology, v. 445, pp. 115-137, 2013; Kims, Chungh, Kongh, et al. Identification of novel immunogenic human Papillomavirus type 16 E7 specific epitopes restricted to HLA-A*b33;03 for cervical cancer immunotherapy.Yonsei Med J, v. 58, p. 43-50, 2017).

The DNA vaccine is more advantageous than the traditional ones due to the simplicity and low cost of production, as it is thermostable dispensing refrigeration, allows repeated doses due to the absence of an immune response against the vector, has the potential to elicit humoral and cellular immunity, and for being highly tolerable in humans [Hancock G, Hellner K, Darrell L. Therapeutic HPV vaccines. Best Practice & Res Clin Obstet Gynecol, 2017. Available at: https://doi.org/10.1016/j.bpobgyn.2017.09.008.

In 2006, the first prophylactic HPV vaccine was approved and released for sale in the United States by the FDA (Food and Drug Administration).

Nowadays, the three HPV vaccines available on the market and released by the FDA and ANVISA (National Health Surveillance Agency) are only prophylactic. Both are based on the immunogenicity conferred to L1 protein when structured in VLPs (virus-like particles).

The first vaccine was licensed in 2006, which is quadrivalent and called Gardasil®, produced by Merck Sharp Dohme, where HPV L1 proteins 6, 11, 16 and 18 are expressed in yeast cells, *Saccharomyces cerevisae.*

The second vaccine was licensed in 2008, which is bivalent and called Cervarix®, produced by Glaxo Smith Kline, consisting of the HPV L1 proteins 16 and 18 that make up the vaccine and are expressed in cultures of insect cells of the SF9 strain.

The third vaccine was licensed in 2014, which is nonavalent and called Gardasil-9®, from the same manufacturer of the homonymous vaccine, but formulated with L1 particles from 9 different types of HPV: 6, 11, 16, 18, 31, 33, 45, 52 and 58 (CDC - Centers for Disease Control and Prevention, available at http://vwvw.cdc.gov/vaccines/he p/vis/vis-statements/hpv-gardasil-9.html.

Currently available vaccines are highly efficient in inducing the production of specific antibodies, that is, they are only prophylactic in nature, and only against the viral types contained in vaccine formulations. Some studies have shown that such antibodies are capable of inducing a small cross-protection against infections by other types of HPV, but still unable to combat the wide range of different types of HPV that cause genital warts and neoplasms.

The Master's Dissertation presented to the Interunit Postgraduate Program in Biotechnology USP/Instituto Butantan/IPT, published in 2009 on behalf of Bruna Felício MM Porchia, entitled: "DESENVOLVIMENTO DE UMA VACINA TERAPÊUTICA PARA TUMORES CAUSADOS PELO VÍRUS PAPILOMA HUMANO TIPO 16" [DEVELOPMENT OF A THERAPEUTIC VACCINE FOR TUMORS CAUSED BY HUMAN PAPILLOMA VIRUS TYPE 16] describes a vaccine strategy against HPV-16-induced tumors, employing a recombinant form of the E7 protein obtained after genetic fusion with Herpes virus type 1 (HSV-1) glycoprotein D (gD). The gDE7 protein generated in a bacterial system was tested as a vaccine in mice, where it conferred 80% prophylactic protection for tumor growth in mice. Soluble gDE7 was able to protect 30% of animals when tested therapeutically.

The aforementioned master's thesis, as well as the present application uses fusion proteins. However, the fusion protein described in said master's thesis is totally different from the fusion protein disclosed by the present application.

Patent application PI 10037497, filed on August 18, 2010 and published on May 2, 2012, in the name of UNIVERSIDADE DE SÃO PAULO - USP and FUNDAÇÃO DE AMPARO À PESQUISA DO ESTADO DE SÃO PAULO - FAPESP and entitled: "PROTEÍNA HÍBRIDA, SEQUÊNCIA DE ÁCIDOS NUCLÉICOS RECOMBINANTE, VETORES/PLASMÍDEOS, FORMULAÇÕES FARMACÊUTICAS E/OU VETERINÁRIAS E SEUS USOS NO CONTROLE DE TUMORES INDUZIDOS PELO VIRUS DO PAPILOMA HUMANO E/OU DOENÇAS INFECCIOSAS OU DEGENERATIVAS" [HYBRID PROTEIN, RECOMBINANT NUCLEIC ACID SEQUENCE, VECTORS/PLASMIDS, PHARMACEUTICAL AND/OR VETERINARY FORMULATIONS AND USES THEREOF IN THE CONTROL OF TUMORS INDUCED BY HUMAN PAPILLOMA VIRUSES AND/OR INFECTIOUS OR DEGENERATIVE DISEASES], formed by the genetic fusion of human papilloma virus type 16 (HPV-16) oncoprotein E7 with a modified form of herpes simplex type 1 (HSV-1) glycoprotein D, deleted in its signal peptide and in the region C-terminal that includes the sequence involved in anchoring the cytoplasmic membrane (corresponding to amino acid sequence 320 to 344), recombinant nucleic acid sequence (SEQ. ID. NO. 1 and SEQ. ID. NO. 2) that encodes the recombinant protein, vectors/plasmids containing the recombinant sequence and pharmaceutical and/or veterinary formulations containing the hybrid protein and/or the recombinant sequence that encodes said protein, preferably, in the form of vaccines. Additionally, the aforementioned patent application is intended for the use of genetic construction, as a vaccine adjuvant to other antigens and/or as an active ingredient in the preparation of pharmaceutical and/or veterinary formulations indicated for the control of tumors induced by human papilloma virus and herpes virus infections.

Patent application PI 10037497, as well as the present application disclose vaccines against HPV. However, the genetic construction described in patent application PI 10037497 is completely different from the fusion protein disclosed by the present application.

Patent application PI 9612675-2, filed on July 29, 1996, published on July 20, 1999, in the name of CANTAB PHARMACEUTICALS RESEARCH LIMITED and entitled "POLIPEPTÍDEO OU COMPOSIÇÃO DE POLIPEPTÍDEO, COMPOSIÇÃO IMUNOGÊNICA ADEQUADA PARA ADMINISTRAÇÃO POR INJEÇÃO, E, USO DE POLIPEPTÍDEO OU DE COMPOSIÇÃO IMUNOGÊNICA" [POLYPEPTIDE OR POLYPEPTIDE COMPOSITION, SUITABLE IMMUNOGENIC COMPOSITION FOR ADMINISTRATION BY INJECTION, AND, USE OF POLYPEPTIDE OR IMMUNOGENIC COMPOSITION] describes fusion polypeptides and polypeptide aggregates comprising antigens derived from *Papillomavirus,* and compositions and use thereof, for example, with adjuvants for vaccine and immunogenic purposes in the generation, for example, of HPV-specific immune responses. Polypeptides can be purified to result in aggregates that, when in solution or dispersion, can pass through a sterilization filter, and in amorphous aggregates. An example of such a polypeptide is a fusion protein of the human *Papillomavirus* L2 and E7 proteins.

With the exception of the L2 fusion process and use, patent application PI 9612675-2 describes a treatment proposal, completely different from the present invention application. Patent application PI 9612675-2 develops an immunotherapeutic with HPV-6 L2 and E7 (non-oncogenic), to combat genital warts, while this application presents HPV-16 L2 and E6 (oncogenic), for prophylaxis and therapy for cervical cancer and other cancers associated with oncogenic HPVs.

The international patent application WO 2017/211886, filed on December 14, 2017 in the name of DEUTSCHES KREBSFORSCHUNGSZENTRUM (DKFZ) and entitled "IMPROVEMENT OF HPV L2 PEPTIDE IMMUNOGENICITY" refers to an immunogenic polypeptide that comprises a multiplicity of peptides Human *Papillomavirus* (HPV) L2 N-terminal corresponding to amino acids 20 to 50 of the HPV16 L2 polypeptide, wherein said HPV L2 N-terminal peptides are L2-N-terminal peptides from at least two different HPV genotypes. Said international patent application WO 2017/211886 also relates to said immunogenic polypeptide for use in medicine and for use in vaccination against HPV infection. In addition, international patent application WO 2017/211886 relates to a polynucleotide encoding the immunogenic polypeptide and a host cell comprising the same.

As can be seen, the international patent application WO 2017/211886, as well as the object of search disclose vaccines against HPV. However, the genetic construction described in international patent application WO 2017/211886 is totally different from the fusion protein disclosed by the present application.

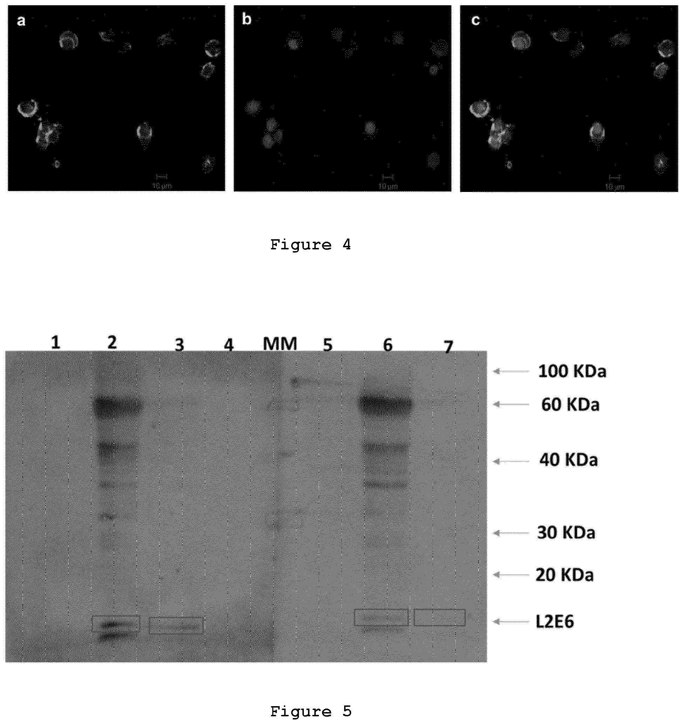

The international patent application WO 2002/38769, filed on September 19, 2001 and published on May 16, 2002, in the name of DEUTSCHES KREBSFORSCHUNGSZENTRUM STIFTUNG DES ÖFFENTLICHEN RECHTS e IPK, INSTITUT FÜR PFLANZENGENETIK UND KULTURPFLANZENFORSCHUNG, entitled "DNA, SEQUENCES, WHICH CODE FOR OPTIMISED EUKARYOTIC HPV 16-L1 AND HPV 16-L2" refers to DNA sequences that have been optimized for codon use, which encode an HPV 16-L1 capsid protein or an HPV 16-L2capsid protein. Said DNA sequences comprise the DNA sequences or fragments or variants thereof which are illustrated in figures 5, 6 or 7 and allow the simple recombinant production of HPV 16-L1 or HPV 16-L2 capsid proteins or fragments thereof with yields without having to use viral vectors. Capsid proteins are preferably used for the production of vaccines.

The international patent application WO 2002/38769, as well as the present application disclose vaccines against HPV. However, the genetic construction described in international patent application WO 2002/38769 discloses an HPV 16-L1 capsid protein or an HPV 16-L2 capsid protein, while the present application discloses an L2/E6 fusion protein, which is totally different from the construction disclosed by the international patent application WO 2002/38769.

As can be seen, no document of the state of the art neither describes nor suggests a therapeutic vaccine against HPV and cancers associated with the virus for human use. Treatments of the state of the art used against injuries and HPV-positive cancers are not specific and depend on the degree of injury. They usually involve treatments with cytotoxic chemical substances, chemical/physical/electrical cauterizations, surgeries, radiotherapies, chemotherapies or combination of treatments, being highly invasive, aggressive and often mutilating.

SUMMARY OF THE INVENTION

In order to solve the aforementioned problems, the present invention will provide significant advantages over the use of an L2/E6 hybrid recombinant protein, thus produced with an effect of inducing the production of specific cytokines such as TNF and anti-L2 and anti-E6 specific antibodies, effectively combating cells transformed by HPV (cancerous or tumor) that express the E6 protein (therapeutic effect), without interfering with healthy body cells (tissue-specific tropism), and new opportunistic viral infections by HPV (broad spectrum prophylactic effect) . Thus, it does not produce side effects, allowing an increase in its performance and presenting a more favorable cost/benefit ratio.

The present invention relates to the development of prophylactic and therapeutic vaccine against HPV and cancers associated with the virus, aimed at people infected by HPV who developed cancer. It refers to DNA expression vectors that can efficiently produce the L2 protein of the HPV16 virus capsid, in addition to the viral E6 oncoprotein associated with human *Papillomavirus* tumors.

Particularly, the present invention relates to obtaining recombinant fusion or hybrid proteins, through gene cloning into expression vectors, produced by the genetic translation of fused genes formed by the combination of nucleic acid regulatory sequences of one or more genes, with the protein coding sequences of one or more genes, used to generate immune responses against HPV.

Specifically, the present invention provides fusions of HPV16 L2 and E6 amino acid sequences, containing only immunogenic epitopes (selected peptides) and not the entire genes, a fact that disables the malignant cell transformation activity (carcinogenesis) of this oncogenic protein, providing security to L2/E6 fusions. In addition, these fusions maintain or increase the immunogenic efficacy of L2 and E6. Considering that the production of more complex recombinant proteins must occur in eukaryotic expression systems (mammalian cells), as is the case therein, to overcome the disadvantages of prokaryotic systems (bacteria) that imply in not making certain post-translational modifications necessary for functional efficiency of the expressed heterologous proteins (eg. glycosylation), also the biological activity may differ from the natural protein; the bacterium has a high content of endotoxins; absence of a secretion mechanism; and there is still the formation of inclusion bodies, whose corrections imply an increase in the final cost of the product.

The hybrid recombinant protein L2/E6, thus produced of the present invention has the effect of inducing the production of cytokines and specific antibodies of anti-L2 and anti-E6 action, effectively combating cells transformed by HPV (cancerous or tumor) that express the E6 protein (therapeutic effect), without interfering with healthy body cells (tissue-specific tropism), and the new opportunistic viral infections by HPV (broad spectrum prophylactic effect) . Thus, it does not produce side effects.

The vaccine developed by the present invention, pcDNA3.3/L2E6, has been shown to be efficient in inducing a long-lasting humoral immune response, capable of stimulating the production of specific anti-L2 and anti-E6 antibodies, in an increasing way and throughout all experimental period (49 days), as well as to activate the cellular immune response, fighting tumor cells and inducing the expression of TNF cytokines.

Therefore, it is worth noting that the present vaccine does not only induce the production of specific antibodies against HPV (prophylactic action), but also induces the production of cytokines, as shown experimentally. Cytokines are important to demonstrate that there is also an induction of responses by cells, which are responsible for fighting tumor cells (therapeutic action). This is the main differential of the present work, in relation to the other works presented herein, since those only induce responses via antibodies.

So far, there is no therapeutic vaccine against HPV and cancers associated to the virus approved for human use in the world. DNA vaccines are stable, easy to produce, transport and store, as they are thermostable and do not require refrigeration. They are able to maintain the expression of the genes of interest, promote the presentation of antigens through MHC Class I and allow the administration of repeated doses, without leading to the production of neutralizing antibodies against the vaccine. The cost is also very low compared to other vaccines. They do not require adjuvants to guarantee their functional efficiency and are highly tolerable in humans.

The vaccine of the present invention is obtained through the techniques of Genetic Engineering: Recombinant DNA Technology, Gene Cloning and Gene Expression of Recombinant Proteins. A vaccine vector was constructed containing peptides selected from HPV16 L2 and E6 proteins, which are expressed in mammalian cells. Amplification of the vaccine vector occurs in bacterial cultures of *Escherichia coli* DH5$\alpha$. The expression of this vector was tested in human epithelial cell lines and in a murine model, in this case to evaluate the efficiency of the vaccine in a prophylactic or therapeutic model, with the mice receiving the vaccine before or after challenge with tumor cells of the TC-1 lineage.

The L2E6 protein expression assays in HEK 293T and 293F cells demonstrated the efficiency of the vector in producing the recombinant protein, demonstrated in indirect immunofluorescence and Western blotting assays.

The animal model tests have shown that the vaccine was able to induce the production of specific anti-L2 and anti-E6 antibodies tested in ELISA, as well as induce the production of TNF cytokines, demonstrated by flow cytometer tests.

BRIEF DESCRIPTION OF THE DRAWINGS

The structure and operation of the present invention, together with additional advantages of the same can be better understood by reference to the attached drawings and the following description:

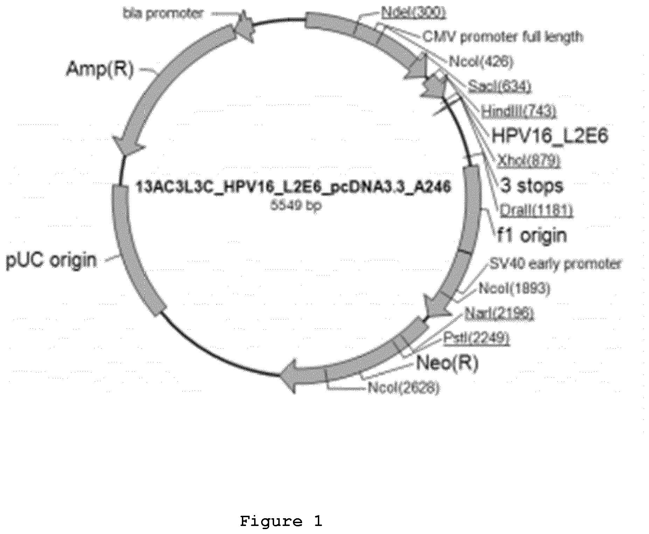

Figure 1 shows the construction map of the expression vector. The L2E6 insert was inserted into the pcDNA3.3 vector flanked by Hind III and Nhe I endonuclease sites, named pcDNA3.3/L2E6. The constructed plasmid originates from bacterial replication, pUC, kanamycin resistance gene, Amp (R), and CMV promoter (Cytomegalovirus). For vector analysis, electrophoresis assays were performed on 0.8% (m/v) agarose gel in 0.5x TAE buffer (40 mM Tris, 20 mM Acetate, 1 mM EDTA) and subjected to an 80V electrophoresis run in the presence of Blue Green Loading Dye (LGC Biotecnologia, Cotia, SP, Brazil).

Figure 2 shows photodocumentation of the agarose gel submitted to electrophoresis for selection of colonies containing the pcDNA3.3/L2E6 vector.

Figure 3 shows Cells transfected with the pcDNA3.3/L2E6 vector expressing the recombinant L2 protein (green - b), the cell nucleus was evidenced by PI (red - c) and in (a) the overlay of the images (b) and (c).

Figure 4 shows E6 Expression (green - a) in 293F cells transfected by pcDNA3.3/L2E6 in an indirect immunofluorescence assay. In (b) it is possible to observe the cell nucleus evidenced by the PI reagent (red). In (c) the overlay of the image (a) and (b).

Figure 5 shows the Photodocumentation of the result obtained from the Western blotting assay used for detection and characterization of the recombinant protein L2E6, where the samples (1) and (5) are aliquots of non-transfected cells lysate, (2) and (6) transfected cells lysate and expressing L2E6, (3) and (7) eluted intermediates obtained from the filtration gel chromatography assay and interacting with specific anti-L2 (3) and anti-E6 monoclonal antibodies, demonstrating the presence of a low molecular weight protein being recognized by both commercial antibodies, (4) final elution sample from the gel filtration process. (MM) Color Burst™ molecular marker.

Figure 6 shows a graphic of the animals' weighing in the DNA vaccine prophylactic evaluation test.

Figure 7 shows a graph of the volume of TC-1 cell tumors in animals previously immunized with the DNA vaccine.

Figure 8 shows a graph of the animals' weight in the therapeutic study of the DNA vaccine of the present invention.

Figure 9 shows a graph of tumor volumes upon treatment with the DNA vaccine of the present invention.

Figure 10 shows the anti-L2 antibody detection assay by ELISA, where the graph of the reactivity of serum samples obtained from the animals on the last experimental day (49 days), demonstrating that all animals immunized with the pcDNA3.3/L2E6 vaccine produced specific anti-L2 antibodies detected by ELISA assay (Cut off = 0.0636) .

Figure 11 shows the anti-E6 antibody detection assay by ELISA, where the graph of serum samples obtained from animals on the last experimental day (49 days), is demonstrating the reactivity of anti-E6 antibodies that all immunized animals produced at the end of the experiment (Cut off = 0.0675).

## DETAILED DESCRIPTION OF THE INVENTION

Although the present invention may be susceptible to different embodiments, it is shown in the drawings and in the following detailed discussion, a preferred embodiment with the understanding that the present embodiment should be considered an example of the principles of the invention and is not intended to limit the present invention to what was illustrated and described in this report.

The present invention relates to a vaccine that is capable of protecting against infection by the HPV virus in a broad spectrum of protection, as well as fighting cells already modified thereby. In particular, this invention relates to obtaining recombinant fusion or hybrid proteins, through gene cloning into expression vectors, produced by the genetic translation of fused genes formed by the combination of nucleic acid regulatory sequences of one or more genes, with the protein coding sequences of one or more genes, used to generate immune responses against HPV. Specifically, this invention provides fusions of HPV16 L2 and E6 amino acid sequences, containing only immunogenic epitopes, a fact that disables the transformation activity of this oncogenic protein, providing safety to L2/E6 fusions. In addition, these fusions maintain or increase the immunogenic efficacy of L2 and E6.

Although humoral immune responses have important preventive utility, several lines of evidence suggest that cell-mediated immune responses play a crucial role in the regression of precancerous lesions and the elimination of infection, of high therapeutic importance. Above all, persistent high-risk HPV infections and pre-invasive lesions are significantly more frequent in immunosuppressed states, such as untreated HIV infection or receiving post-transplant anti-rejection therapy.

Spontaneous regression of high-grade CIN (Cervical Intraepithelial Neoplasm) has been reported to correlate with cell-mediated immune responses. Regression lesions are typically infiltrated by CD8 + T cells specific for viral oncoproteins, E6 and E7.

In addition, CD4 + T cell responses to E2, a key transcriptional regulator, were detected in individuals after CIN regression. Increased proportions of CD4:CD8 in the stroma have also been reported. In contrast, reduced numbers of CD4 + T cells are present in persistent and/or progressive CIN lesions. Regulatory T cells (Tregs) are also found in persistent HPV infections, with increasing frequency with the size of genital warts.

After administration, the DNA vaccine encoding the hybrid L2/E6 proteins will express them in the patient's tissues. The hybrid recombinant protein L2/E6 thus produced has the effect of inducing the production of cytokines and specific antibodies with anti-L2 and anti-E6 action, effectively combating cells transformed by HPV (cancerous or tumor) that express the E6 protein (effect therapeutic), without interfering with healthy body cells (tissue-specific tropism), and new opportunistic viral infections by HPV (broad spectrum prophylactic effect). Thus, it does not produce side effects.

### 1. Selection of epitopes and construction of vectors

The sequences of the HPV16 L2 and E6 genes were obtained from the international database GenBank (r), and the access code of the E6 gene used was AAD33252 and the reference for L2 access was NC_001526.2. From the sequences of the complete genes, peptide sequences were selected according to their ability to induce an immune response described in other studies.

The peptides selected from the E6 gene are among the amino acid residues 50 to 57 (YDFAFRDL) (SEQ ID NO: 5), responsible for inducing cellular immune response through the activation of cytotoxic T lymphocytes.

The codons were optimized for greater success in their expression in human cells, using the Codon Optimization tool from IDT - Integrated DNA Technology, and the final sequence obtained was TATGATTTCGCCTTCAGGGACCTC (SEQ ID NO: 4).

For the L2 gene, the selected peptide was found among amino acid residues 17 to 36 (QLYKTCKQAGTCPPDIIPKV) (SEQ ID NO: 2), the sequence being optimized as described above, resulting in: CAGCTGTATAAAACTTGTAAGCAGGCCGGTACGTGCCCCCCAGATATCATTCCAAAGGT C (SEQ ID NO: 3).

Both sequences were linked by 3 "Linker" sequences, resulting in the 96 bp sequence (base pairs), to which the initiator and termination sequences were added, giving rise to the following 102 bp sequence: ATG CAG CTG TAT AAA ACC TGT AAA CAG GCA GGT ACA TGT CCG CCG GAT ATT ATT CCG AAA GTT GGT GGT AGC GGC TAT GAT TTT GCC TTT CGC GAT CTG TAA. (SEQ ID NO: 1). The final sequence was synthesized by the company Gene Art™ (Thermo Fisher Scientific) giving rise to the synthetic gene called L2E6.

### 2. Construction of vectors

2 vectors were designed containing the synthetic gene L2E6 and developed by the company Gene Art™ (Thermo Fisher Scientific), being a cloning vector based on the commercial vector pMAT (Thermo Fisher Scientific) and an expression vector or vaccine vector in pcDNA (Thermo Fisher Scientific) which will be better described below.

For the construction of the cloning vector, the synthetic gene L2E6 was inserted in pMA-T, with both ends of the synthetic gene and the vector flanked by endonuclease Sfi I sequences (Thermo Fisher Scientific).

The vector called pMA-T/L2E6 has bacterial Col E1 replication origin and kanamycin resistance gene, AmpR. The complete vector has 2494 bp.

For the construction of the expression vector, the L2E6 insert was inserted into the pcDNA3.3 vector flanked by Hind III and Nhe I endonuclease sites, receiving the name pcDNA3.3 / L2E6. The constructed plasmid originates from bacterial replication, pUC, kanamycin resistance gene, Amp (R), and CMV promoter (*Cytomegalovirus*).

For vector analysis, electrophoresis assays were performed on 0.8% (m/v) agarose gel in 0.5x TAE buffer (40 mM Tris, 20 mM acetate, 1 mM EDTA) and subjected to running 80V electrophoresis in the presence of Blue Green Loading Dye (LGC Biotecnologia, Cotia, SP, Brazil).

### 3. Chemiocompetence of bacteria

For the amplification of the vectors, bacterial strains *Escherichia coli* DH5a and TOP10 were used. The bacterial strains were cultured in 3 mL of Luria-Bertani (LB) medium (2% tryptone, 1% yeast extract, 1% NaCl) at 37°C, under agitation at 250 rpm for approximately 18 hours.

Then, an aliquot of this culture was streaked in a Petri plate containing LB medium plus 1.5% agar, incubated at 37°C, for approximately 18 hours.

The next day, a colony was chosen to sow 3 mL of LB medium and grown under the same conditions as previously described. After that period, 1 mL of this culture was used to sow 100 mL of LB medium and grown at 37°C, under agitation until the optical density of the culture at 600 nm (DO600) reached 0.4, wherein the cultured bacteria were in half exponential growth phase.

Competence induction was performed according to a protocol established by Sambrook et *al.* (2001), where after reaching the desired cell density, the bacterial culture was transferred to a conical tube and centrifuged at 5000xg for 10 minutes and incubated in a 0.1 M $CaCl_2$ solution for 1 hour on ice. Then, the bacteria were again centrifuged and resuspended in a 0.1 M $CaCl_2$ solution plus 10% glycerol (w/v).

200 $\mu$L aliquots were frozen at -80°C until use.

### 4. Bacterial transformation

From the competent bacteria described above, bacterial transformation was carried out, wherein a mixture of 10 $\mu$L of plasmideal DNA, approximately 15 g, and an aliquot of chemocompetent bacteria was incubated for 30 minutes on ice, subjected to a thermal shock for 2 minutes at 42°C and incubated again on ice for 5 minutes. Then, 350 $\mu$L of SOC medium (2% tryptone, 0.5% yeast extract, 10 mM NaCl, 2.5 mM KCl, 10 mM MgCl2, 10 mM MgSO4, 20 mM glucose) were added, and the mixture was incubated for 90 minutes at 37°C, under agitation of 250 rpm. Then, the transformed bacteria were seeded on plates of LB-agar medium containing or not 100 $\mu$g/ml kanamycin and incubated at 37°C for 18 hours.

Colonies grown on LB-agar plates containing kanamycin identify the positive clones that were selected by colony PCR and seeded in tubes containing 3 mL of LB medium, incubated at 37°C, under agitation for approximately 18 hours.

## 5. Selection of bacterial colonies

For selection of bacterial colonies after transformation, the colony PCR technique was used, where using specific primers synthesized from the L2E6 gene sequence, 2 primers were designed, EAK03 and EAK02, and synthesized by the company EXXTEND Biotecnologia Ltda. (Campinas, SP, Brazil), capable of generating a 144 bp product. The sequence of the primers can be seen below.

EAK03 - CCCYYAAGCTTGCCACCATGCAGC (SEQ ID NO: 6)
EAK02 - GAGTGTCTAGATGCCACGCT (SEQ ID NO: 7)

The primers were resuspended in TE buffer (10 mM Tris/HCl, 1 mM EDTA) at a stock concentration of 100 $\mu$M.

For PCR, each solution was prepared separately in microtubes containing 25 $\mu$L of PCR-Mix solution (1.5 mM $MgCl_2$, 0.5U Taq, 200 5M dNTPs - LGC Biotecnologia, Cotia, SP, Brazil), 0.5 $\mu$L of each initiator at 25 $\mu$M and ultrapure water for a final solution volume of 50 $\mu$L. As a template DNA, a sample of each colony of transformed bacteria was used, selected and grown in LB medium with kanamycin. Amplification in a Mastercycler EpGradient thermocycler (Eppendorf, Germany) occurred following the protocol: 95°C for 5 minutes, 25 cycles of 1 minute at 95°C, 30 seconds at 56°C and 1 minute at 72°C, followed by final extension at 72°C for 7 minutes.

The detection of PCR products occurred through electrophoresis in 0.8% agarose gel in 0.5X TAE buffer. The samples were prepared with the addition of 0.5 $\mu$L of Blue Green Loading Dye I and subjected to a run at 80 A for 30 minutes. As a parameter for fragment size, 2 molecular weight markers were used, the Universal Marker containing 8 fragments from 10000 bp to 100 bp (LGC Biotechnology) and the second 50 bp DNA Ladder marker containing 12 fragments from 1000 bp to 50 bp (LGC Biotechnology). The gel was analyzed in the High Perfomance Ultraviolet Translluminator (UVP Ultraviolet Products, Upland, USA) and the images captured in Alliance 9.7 photo-documenter (UVITEC Cambridget, Cambridget, England).

## 6. Plasmid DNA extraction

The positive clones selected by the colony PCR were cultured in 5 mL of LB medium, at 37°C, for approximately 18 hours, under agitation, in the presence of the antibiotic of selection. The next day, they were centrifuged at room temperature, for 1 minute at 12000xg to sediment the intact bacteria. For the purification of plasmideal DNA, the GeneJet Plasmid Miniprep Kit (Thermo Fisher Scientific) was used, which is based on the alkaline lysis procedure combined with a selective binding column to the circular DNA, performed according to the manufacturer's instructions. The purified DNA was analyzed on 0.8% agarose gel in 0.5X TAE buffer and subjected to the sequencing technique to confirm the insertion of the gene of interest to the vector, quantified in a spectrophotometer (ACTGene, New Jersey, USA) and stored at -20°C.

## 7. DNA sequencing

Plasmid DNA samples were sequenced to confirm the correct insertion of the L2E6 gene and its integrity. For this purpose, the DNA samples purified from the gel as described in the previous session were quantified by spectrophotometry. A volume of 5 $\mu$L of the plasmid was aliquoted and 2.5 $\mu$L of the EAK03 primer was added at a concentration of 5 $\mu$M. The samples were sent to the Human Genome Center, at the University of São Paulo, where the sequencing reaction took place on the ABI 3730 DNA Analyzer (Thermo Fisher Scientific).

## 8. Cell lines and cultivation conditions

At first, to check the expression of the pcDNA3.3/L2E6 vector, the transfection process was performed on HEK293T cells cultured in Eagle medium modified by Dulbecco (DMEM, Cultilab, Campinas, SP, Brazil) supplemented with 10% fetal bovine serum (SBF, Cultilab, Campinas, SP, Brazil), D10, kept in a humid atmosphere, containing 5% $CO_2$ until reaching 50-80% confluence. The raising occurred every 3 or 4 days.

The cell line chosen for the expression of recombinant proteins on a larger scale was the 293F, Freestyle™ 293 Expression System (Life Techonologies™), which was developed to allow large-scale transfection of cells suspended in a defined and serum-free medium.

293F cells were cultured in 30 mL of Freestyle™ 293 Expression medium (Life Technologies™) in sterile glass flasks at 37°C and 5% $CO_2$, in a humid atmosphere, under agitation with rotation at approximately 135 rpm. Cell replays were performed every 3 or 4 days, with cell density of approximately 2 to $3\times10^6$ viable cells/mL, where the cells were transferred to conical tubes and centrifuged at 800 rpm for 5 minutes. Then, cell viability was analyzed and the cells diluted to a final cell density of $3\times10^5$ viable cells/mL in fresh preheated medium.

Trypan blue exclusion method (0.4% in PBS) and counting in a Neubauer chamber were used to determine cell viability.

9. Cell transfection

The transient transfection assay of HEK 293T cells was carried out by lipofection using the GeneJuice® Transfection Reagents (EMD Biosciences/Merck, Darmstadt, Germany), according to the manufacturer's specifications. Briefly, the transfection reagent was mixed by vortex to an aliquot of DMEM medium, being incubated for 5 minutes at room temperature. The plasmideal DNA of interest was added to the mixture, in the appropriate proportion, gently homogenized and incubated for 15 minutes at room temperature. Then, the Medium/transfection reagent/DNA mixture was added to the cells with complete culture medium, and incubated in an oven at 37°C and 5% $CO_2$. After the 8-hour period, the culture medium was changed, now with a complete medium containing antibiotics and 10% SBF. The incubation continued for approximately 48 hours when the cells were collected and analyzed in immunofluorescence and in SDS-PAGE electrophoresis gel, which will be described in later topics.

For 293F cells, transfection assays were performed according to the manufacturer's instructions, using 293fectin transfection reagent.

One day before transfection, the cell density of viable 293F cells was determined and adjusted. In 28 mL of Freestyle™ Expression Medium, $7 \times 10^5$ cells were added, cultured under conditions described above.

The next day, a small aliquot of the cell culture was transferred to a microtube to determine the cell viability rate, which should remain around 90%. For the transfection of 3x107 viable cells, two solutions were prepared in different sterile conical tubes:

Tube 1 - 30 μg of plasmideal pcDNA3.3 / L2E6 DNA in 1 mL of final volume with Opti-MEM® medium, gently homogenized;

Tube 2 - 60 μL of 293fectin™ in 1 mL of final volume with Opti-MEM® medium, mixed gently and incubated for 5 minutes at room temperature.

Then, the contents of tubes 1 and 2, described above, were mixed gently resulting in a 2 mL solution, which was incubated for 30 minutes at room temperature, to allow plasmideal DNA to form complexes with 293fectin® reagent.

While the DNA-293fectin® complex was incubated, the cells in culture were centrifuged in conical tubes at 800 rpm for 5 minutes, with the medium in culture being replaced by 28 ml of fresh, preheated Freestyle™ Expression Medium.

Finally, the 2 mL of the DNA-293fectin® complex was added to 28 mL of medium and cells in suspension, totaling 30 mL of solution for transfection, incubated 48 hours at 37°C and 5% $CO_2$, in a humid atmosphere, under shaking in an orbital shaker at approximately 125 rpm.

10. Expression and characterization of recombinant proteins

10.1. Intracellular expression of proteins

After transfection, an aliquot of the cells was separated for analysis of the expression of the intracellular recombinant proteins through immunofluorescence assays.

HEK 293T cells were cultured on 13 mm diameter round coverslips in 12 well culture plates (TPP®). The easily adherent strains were grown directly on the coverslips in D10 medium, kept in a humid atmosphere, containing 5% $CO_2$, for approximately 18 hours. In the following day, the cell transfection assay was performed as described in the previous item.

For assays using 293F cells already transfected, as previously described, they were centrifuged in microtubes and the supernatant discarded.

After the transfections, both cell lines were washed 2 times in PBS and fixed in a 1% paraformaldehyde solution in PBS, for 1 hour at 4°C. After successive washes to remove all fixative, the cells were incubated in a PBS+BSA 5% solution to block nonspecific bonds and incubated for 1 hour at room temperature. Then, the cells were washed in PBS for 3 times and then incubated with specific primary anti-HPV16 L2 or anti-HPV16 E6 antibodies diluted in 0.5% BSA and 0.05% Tween 20 in PBS for 1 hour at room temperature, shaking the tube frequently. After washing again, the cells were incubated with the respective specific secondary antibodies diluted in 1.5% BSA and 0.01% Tween 20 in PBS for 1 hour at room temperature, with frequent shaking in the tube containing the cells and the solution with the antibodies. The secondary antibodies used were AlexaFluor® 488 anti-mouse IgG produced in goat (Molecular Probes®, OR) and AlexaFluor® 633 anti-rabbit IgG produced in goat. After a final wash in PBS, 293F cells were distributed on glass coverslips coated with 0.5% poly-L-lysine in PBS.

Finally, the coverslips containing HEK 293T or 293F cells were mounted on slides with Mowiol (Calbiochem, CA, USA), containing propidium iodide (PI - Molecular Probes®, OR) that evidence the presence and location of nucleic acids.

The preparations were kept at 4°C, protected from light until the moment of their analysis in the Zeiss LSM 510 Meta laser-scanning confocal microscope.

*10.2. Analysis of the expression of recombinant proteins*

After each transfection reaction, cells were lysed in buffer (0.5 M Tris pH 7.4, 0.5 M NaCl, 0.5 M EDTA pH 8.0, 0.2 M EGTA, Triton X-100, protease inhibitor cocktail), centrifuged at 10,000xg for 5 minutes, with the precipitate discarded and the supernatant stored at -20°C until the time of use.

In order to confirm the transfection, the samples of cell lysates were analyzed in a faster and simpler way through dot blotting assays. Thus, 10 μL of samples were exposed to nitrocellulose membrane strips (Bio-Rad Laboratories, Inc.). After drying, the strips were incubated in a solution containing 5% skimmed-milk powder in PBST for 1 hour, under agitation at room temperature. Then the membrane strips were washed 4 times in PBST, for 10 minutes, and incubated with anti-HPV16 L2 (Santa Cruz Biotechnology, Inc.) or anti-HPV16/18 E6 (Abcam®) monoclonal antibodies diluted in 1% skimmed-milk powder in PBST for 1 hour at room temperature, under stirring. Then, the strips were washed again for 10 minutes in PBST, 4 times, and incubated in secondary anti-mouse IgG antibody conjugated to horseradish peroxidase (Sigma-Aldrich®) diluted in PBST and 1% skimmed milk powder, for 1 more hour, stirring at room temperature. Then, the samples were washed for another 4 times of 10 minutes in PBST and the development was done by a DAB substrate solution (diaminobenzidine) - peroxidase (0.05% DAB, 0.015% $H_2O_2$, 0.01 M PBS, pH 7.2), incubated at room temperature for up to 10 minutes, under agitation and protected from light.

The positive samples in the dot blot assays were taken for analysis of the expression of recombinant proteins, carried out through Western blotting assays. For this purpose, a 16% Tricina-SDS-PAGE gel (Sodium Dodecyl Sulphate Polyacrylamide Gel Electrophoresis) was prepared and submitted to electrophoretic separation (30 V for 300 minutes), under denaturing conditions, containing samples of transfected cell lysates diluted in buffer of racing. Then, the gel was stained in Coomassie Blue solution (7% acetic acid, 50% Methanol, 0.1% Coomassie Brilliant Blue R-250) or the proteins were transferred to a nitrocellulose membrane for Western blotting assays. After electrophoretic transfer (30 V and 15 mA for 18 hours, or 70 V and 35 mA for 2 hours) in transfer buffer (0.025 M Tris, 0.192 M Glycine, 20% Methanol) at 4°C, the membrane was stained in a solution containing Ponceau temporary dye (Ponceau S 0.1%, acetic acid 5%) to confirm the transfer and marking of molecular weight indicators. Then, the membranes were washed in distilled water to remove temporary staining and incubated in a solution containing 5% skimmed-milk powder diluted in PBST (Tween 20 0.05%, PBS), for 1 hour, at room temperature, under agitation. After that period, the membranes were washed 4 times in PBST and incubated with 1% skimmed-milk powder solutions in PBST containing the primary antibodies anti-HPV16 L2 monoclonal, anti-HPV16 L2 produced in rabbit (kindly provided by Prof Dr RBS Roden, John Hopkins School of Medicine, USA) and/or anti-HPV16 E6 for 2 hours at room temperature, under stirring. Then, the membranes were again washed 4 times in PBST and incubated in a solution containing the respective secondary anti-mouse IgG or anti-rabbit IgG antibodies conjugated with horseradish peroxidase in 1% skimmed-milk powder solution in PBST, incubated for 2 hours at room temperature, under agitation. The reaction was detected using the Novex® ECL HRP Chemiluminescent Substrate Reagent (Invitrogen) kit on green light sensitive films (Kodak).

11. Protein analysis

The samples of cell lysate were analyzed on 16% Tricina-SDS-PAGE electrophoresis gel, the most suitable for the separation of proteins from 1 to 100 KDa (SCHAGGER, 2006) .

For this purpose, the 16% separation gel was first prepared, where the AB-6 buffer (acrylamide 46.5 g, bis-acrylamide 3 g) was diluted in gel buffer (Tris 3 M, HCl 1M, 0.3% SDS, pH 8.45), plus 10% PA [$(NH_4)\,2S_2O_8$] and TEMED [$(CH_3)2NCH_2CH_2N(CH_3)_2$]. The gel was quickly placed in the apparatus to polymerize. Then, a new 10% separation gel was prepared, using AB-3 buffer (48 g acrylamide, 1.5 g bis-acrylamide), diluted in the same gel buffer, water, 10% PA and TEMED. After preparation, the 10% gel was gently placed on the 16% gel. Finally, a stacking gel was prepared, where the AB-3 buffer was diluted in gel buffer, water, 10% PA and TEMED to a final concentration of 4%.

Protein samples were quantified and the concentrations adjusted so that each sample contains approximately 15 μg of proteins. Protein dosage was performed using the Bradford method (Bio-Rad Laboratories Inc.), according to the manufacturer's instructions. Thus, the samples were diluted in sample buffer (Tris-Cl 1 M pH 6.8, SDS 0.8%, bromophenol blue 0.1%, glycerol 40%, b-mercaptoethanol 14.4 M) and finally submitted to electrophoretic separation (30 V for 300 minutes) in tris-tricine buffer (0.1 M Tris; 0.1 M Tricine; 0.1% SDS, pH 8.5). After separation, the gel was stained with Coomassie blue solution.

12. Peptide synthesis

To be used as positive controls of the peptides designed in the present study, synthetic peptides L2 (PEP2) and E6 (PEP1) were requested from FastBio Ltda. (Ribeirão Preto, SP).

PEP1, refers to the selected peptide of the HPV16 E6 protein, whose sequence YDFAFRDL, containing 8 amino acids and purity> 85%.

PEP2, selected from the HPV16 L2 protein, has the sequence LYKTCKQAGTCPPDIIPKV, with 19 amino acids and purity > 85%.

The peptides were received lyophilized and were resuspended in ultrapure water at a concentration of 5 mg/mL.

13. Cultivation of TC-1 tumor cells

Primary lung cells from C57BL/6 mice were transduced with the HPV16 E6 and E7 genes, then the immortalized cells were again transduced with the activated human c-Has-ras gene, generating the stable transformed cell line TC-1 that express the HPV16 protein E6 and E7, being able to generate solid tumors in mice with the same genetic background (LIN, 1996).

TC-1 cells were cultured in 75 $cm^2$ polystyrene flasks (TPP - TPP Techno Plastic Products AG, Switzerland) in RPMI medium (Cultilab, Campinas, SP, Brazil), supplemented with 10% fetal bovine serum (SBF) (Cultilab, Campinas, SP, Brazil) at 37°C and 5% $CO_2$. The cells were replanted or the culture media changed every 3 or 4 days. For raising, trypsin / EDTA 250 mg% solution (Cultilab) was used to remove the cells adhered to the bottom of the culture flasks, incubated for up to 15 minutes, at 37°C. Then, the cells were centrifuged at 800xg for 15 minutes at 4°C. The supernatant is discarded and the cells resuspended in new culture medium. For cell line storage for long periods, aliquots of the cells were stored in a solution of RPMI culture medium containing 10% SBF, plus 10% DMSO, in liquid nitrogen.

14. *In vivo* experiments

In order to evaluate the possibility of using the vectors constructed as vaccine vectors, since the chosen plasmid, pcDNA, also allows such use, *in vivo* tests were performed.

For animal tests, the present invention was analyzed and approved by the Animal Use Ethics Committee of the Butantan Institute, under protocol number 923/12.

C57BL/6 male mice of 4 to 8 weeks provided by the Central Animal Hospital of the Butantan Institute, with an average weight of 20 g, were separated into 4 groups containing 10 animals in each group, kept in a vivarium with food and water *ad libitum* and controlled light cycle.

The animals were weighed weekly before and throughout the experiment.

14.1. *Prophylactic evaluation*

Two groups of 4 or 6 animals were selected for prophylactic evaluation of the proposed DNA vaccine.

The first group, called group I, containing 4 animals received 50 μL of intramuscular PBS solution in 3 doses in the interval of 14 days between the first (day 1) and second dose (day 14), and 7 days (day 21) between the second and third doses.

The other 6 animals that made up group II, received 3 doses of the vaccine construct pcDNA3.3/L2E6 in PBS solution, in decreasing concentrations. The first dose occurred on day 1 and the animals received 50 μg of the DNA vaccine. The second dose was administered 14 days later, at a concentration of 20 μg, while the third and last dose presented a concentration of 5 μg of the vaccine vector, applied 7 days after the previous dose.

The vaccines were administered intramuscularly in the anterior tibial muscle of the hind leg, and the doses were administered intercalating between the right and left hind legs. The inoculated volume never exceeded 50 μL of solution.

Seven days after the last dose (day 28), all animals in both groups were challenged with $10^5$ TC-1 cells in PBS solution via the subcutaneous route in the left dorsolateral region of each animal. The mice were observed daily and 8 days after the implementation of the tumor cells it was possible to detect the formation of a palpable mass. From the detection of the tumors, they were measured every 2 days with the aid of a digital caliper (Western®, China). The tumors grew in irregular shapes and were measured for length and the perpendicular axis, width, in the same plane. From these data, the tumor volume of each mouse in $cm^3$ was calculated, the data were grouped and analyzed statistically using Student's t-tests (p = 5.00%), and the Graphic-Pad Prism 6 software, version 3.0.

Small blood aliquots, approximately 100 μL, were collected from all animals on days 0, 14, 28, 42. The mice were euthanized about 3 weeks after the implantation of the tumors (day 49), following the instructions ethical standards in animal experimentation (TAMBOURGI et *al.,* 2010) and all blood was collected.

From the blood samples, the animals' serum was collected by centrifugation at 800 rpm, where approximately 30 μL of serum were obtained and stored at - 20°C until the time of analysis.

*14.2. Therapeutic evaluation*

To evaluate the efficiency of the use of the constructed vaccine vector, pcDNA3.3/L2E6, in the treatment of cancers associated with HPV, 3 animal groups were selected for the trials.

The groups were formed by 10 animals and challenged with $7 \times 10^4$ TC-1 cells in DMEM culture medium and inoculated subcutaneously in the left dorsolateral region of each animal. Previously, the area was prepared by shaving the hair to facilitate the application of tumor cells by the correct route.

The day after tumor cell inoculation, day 1, the animals in the control group, group III, received 5 μg of the empty vector, pcDNA3.3, and on day 10, a second dose containing 5 μg of the same vector was administered.

The animals were monitored daily and the region was palpated to check the tumor implantation. As soon as the tumors were detected, they were measured manually with the aid of a digital caliper every 2 days, or until the tumors reached approximately 20 mm in diameter, when the animals were euthanized.

For the experimental tests, the animals were divided into 2 new groups, where the first group, group IV, received the first dose of the DNA vaccine, pcDNA3.3/L2E6, on the first day of experimentation, and the second dose, at 15 days. The first dose with 5 μg of vector pcDNA3.3/L2E6, while the second dose was composed of 5 μg of the vaccine vector. These animals also received a third vaccine dose of 5 μg at 28 experimental days. Both doses were diluted in PBS solution.

Animals from the second experimental group, group V, received the first dose of the DNA vaccine containing 5μg of pcDNA3.3/L2E6 on the first experimental day, that is, 1 day after receiving the tumor cells, and the second dose 10 days after the first one containing 5 μg.

The animals were monitored daily and the region was palpated to check tumor growth. If detected, the tumors would be measured manually with the aid of a digital caliper every 2 days, or until the tumors reached approximately 20 mm in diameter, when the animals would be euthanized.

Small aliquots of blood were taken on days 0, 21, 28 and 49 to obtain serum and stored at -20°C until the time of use.

The spleens of the animals that were part of the groups tested for the therapeutic action of the DNA vaccine, groups III, IV and V, were collected in a sterile environment, washed twice in sterile PBS solution and cultured as described below.

15. Splenocyte culture

The spleen removed from each animal, after double washing in sterile PBS solution, was macerated against a piece of sterile gauze with the aid of a syringe plunger in a Petri plate. 3 ml of RPMI culture medium supplemented with 10% fetal bovine serum (R10) was added to the macerate. The cells were collected in a sterile conical tube and centrifuged at 12000 rpm for 5 minutes.

After centrifugation, the supernatant was discarded and the red blood cells were lysed by adding 500 μL of sterile distilled water, homogenized quickly and adding another 500 μL of PBS solution twice concentrated and sterile, homogenizing well. After another centrifugation at 1000 rpm for 10 minutes, the supernatant was again discarded and the precipitate was resuspended with 1 mL of R10 medium.

Viable spleen cells were counted with the help of trypan blue dye in 0.4% solution in PBS in a Neubauer chamber.

The cells were distributed in triplicate in a 12-well culture plate at a concentration of $10^6$ cells/ml, the volume being made up to 1 ml of R10 medium. 10 μL of the synthetic peptide PEP1 (E6), described above, was added to each well. Then, the plates containing the splenocytes and PEP1 were incubated for 48 hours at 37°C and 5% $CO_2$, in a humid environment. After this period, the culture supernatant was recovered and stored at -80°C until the moment of cytokine dosage analyzes, described below.

16. Cytokine detection assay

From serum aliquots collected from the animals under experiment and from the splenocyte culture supernatant, dosages of soluble cytokines were performed with the aid of the BD CBA Mouse Th1/Th2 Cytokine kit (BDTM Biosciences, USA) according to manufacturer specifications.

Briefly, the samples were separated by groups and experimental dates, being analyzed in pools only the serum samples collected on days 0, 28 and 49, as well as the samples of the splenocyte culture supernatant. The samples were incubated with beads coated with specific antibodies against 5 different types of cytokines (IL-2, IL-4, IL-5, INF-g and TNF), together with the detection reagent conjugated to fluorescent protein PE (Ficoeritrina) and incubated for 2 hours at room temperature, protected from light.

Then, the beads were washed with washing buffer (kit component) and resuspended in 300 μL of washing buffer, being then analyzed in a flow cytometer, BD FACSCanto II (BD™ Biosciences) . The data were analyzed using FCAP Array software version 3.0 (BD™ Biosciences).

17. Indirect ELISA

For analysis of the induction of humoral immune response by the pcDNA3.3/L2E6 vaccine, ELISA assays (Enzymed-Linked ImmunoSorbent Assay) were performed, where the flat-bottom plates with high adhesion capacity (Sarstedt, Numbrecht, Germany) were sensitized with 0.6 mg/ml PEP1 or 0.3 mg/ml PEP2 in carbonate-bicarbonate buffer (0.2 M $Na_2CO_3$, 0.2 M $NaHCO_3$, pH 9.6) and incubated for 18 hours at 4°C. The next day, the antigen was recovered and the plate was washed 3 times with PBST. Then, 150 $\mu$L of PBST solution + 5% skimmed-milk powder were added to block nonspecific bonds and incubated for 1 hour at room temperature. The plate was washed again 3 times with PBST and in each well the serum samples collected from the animals were distributed on days 0, 28 and 49, diluted 1:100 in PBST solution + 1% skimmed milk powder. The primary antibodies were incubated for 2 hours at room temperature. After this period, the samples were discarded and the plate was washed 3 more times with PBST solution. Then, 100 $\mu$L of secondary antibody, anti-mouse IgG produced in sheep conjugated to peroxidase (Sigma-Aldrich), diluted 1:250 in PBST solution + 1% skimmed milk powder and incubated for 1 hour and 30 minutes at room temperature. The plate was washed again 3 times with PBST solution and 1 time with PBS. Finally, each well received 100 $\mu$L of developer solution (0.05M $C_6H_8O_7$, 0.05M $Na_2HPO_4$, pH 5) in which an OPD tablet (o-phenylenediamine dihydrochloride, Invitrogen) containing 5 mg and 12 $\mu$L of $H_2O_2$ was added to 30%. Incubated for 15 minutes at room temperature in a place protected from direct light. Then, each well received 50 $\mu$L of 2.5 M $H_2SO_4$ solution to stop the reaction. The plate was then read on a MultiSkan EX Reader (LabSystems) at 492 nm. The samples were tested in triplicate.

The analysis of the results was performed by calculating the cut-off point ("cut off") by the average of the negative samples plus 3 times the value of the standard deviation(s) of these samples, with an additional 10% of the final value. The number of standard deviations used in the formula guarantees the confidence level of the result.

```
Cutoff point = average + 3x s + 10%
```

All points obtained above this cutoff point were considered to be positive samples.

18. Statistical analysis

Statistical analyzes were performed with the aid of the GraphPad Prism® software (GraphPad Software, Inc.). Student's t tests (p = 0.05%) were used for statistical analysis on data obtained in animal experiments, animal weight and tumor volume. In the cytokine detection tests, the ANOVA parametric test (p <0.05) was used.

19. Bioinformatics analysis of the L2E6 sequence.

From the DNA sequence coding for the defined L2E6 protein, *in silico* analyzes were performed to better understand the product to be generated and the characteristics of the protein to assist the development of the present study.

Table 1 shows the bioinformatics analyzes of the L2E6 sequence. From the DNA sequence coding for the defined L2E6 protein, *in silico* analyzes were performed to better understand the product to be generated and the characteristics of the protein to assist in the development of the present study. Several software and online sites for proteomic analysis have emerged in recent years, based on algorithms that predict protein characteristics from their amino acid sequences. One of the most used and described sites in the literature capable of predicting the physicochemical characteristics of proteins is http://protcalc.sourceforge.net/, which estimates molecular mass, pH, pI and performs the counting of amino acid residues. Several reference sites were consulted and the data were analyzed and compiled in the table below, where the defined sequence QLYKTCKQAGTCPPDIIPKVGGSGYDFAFRDL with 32 amino acid residues showing the other characteristics:

Table 1 - Physico-chemical properties of the L2E6 protein drawn in the present study, according to the Protein Calculator website (http://protcalc.sourceforge.net/).

| Physico-chemical properties | |
|---|---|
| Molecular mass | Approximately 3.5 KDa |
| pI | 7.98 |
| Estimated load at pH 7.0 | 0.9 |

Using other prediction sites for possible post-translational changes, several parameters were analyzed through the website of the Center for Biological Sequence Analysis - CBS - of the Technical University of Denmark through the link http://www.cbs.dtu.dk/index.shtml.

The analyzes demonstrated that the developed L2E6 peptide presents prediction sites for acetylation, phosphorylation, leucine-rich sites indicating signs of export to the nucleus and a cleavage site between amino acid residues 23 and 24, generating a signal peptide with possible signaling of export of this protein to the transmembrane region. In addition to the presence of 2 cysteine amino acid residues, which can lead to the formation of disulfide bonds with post-translational modifications generating possible changes in the conformation and stability of the protein.

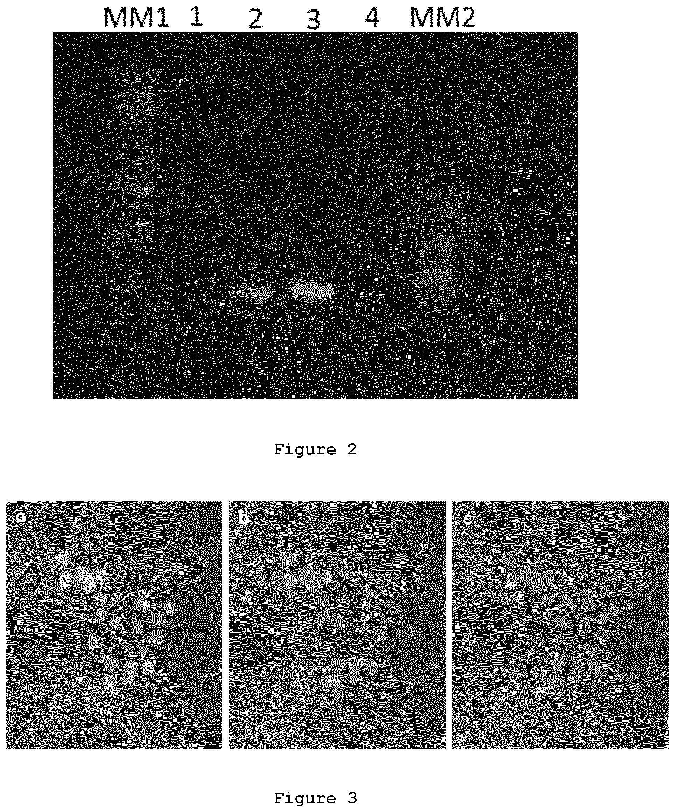

Figure 2 shows the photodocumentation of electrophoresis gel in 0.8% agarose for selection of bacterial colonies after transformation with the pcDNA3.3/L2E6 vector. After the bacterial transformation assays to amplify the vectors, the bacterial colonies were submitted to PCR to select the positive clones. The PCR products were analyzed on an agarose gel and subjected to electrophoresis (Figure 2), where (MM1) represents the universal marker and (MM2) the 50 bp DNA Ladder marker. In (1) we have a sample of the intact plasmid pcDNA3.3/L2E6, in (2) and (3) PCR products from bacterial colonies transformed by pcDNA3.3/L2E6 and (4) PCR product from an untransformed colony.

Thus, the colony presented in column 3 (Figure 2) was selected for amplification of the bacterial colony and then for the purification of plasmideal DNA. The recovered plasmideal DNA was quantified by spectrophotometry, resulting in an average of 150 ng/$\mu$L per sample.

The sequencing of the plasmideal DNA recovered and quantified as previously described confirmed the correct formation and sequence of the L2E6 gene developed in the present work.

Figures 3 and 4 show the evaluation of the intracellular expression of the recombinant fused protein L2E6, performed in HEK293T (Figure 3) and 293F cells (Figure 4) .

Through indirect immunofluorescence assays it was possible to evidence the expression of the recombinant protein, being recognized by the specific anti-L2 commercial monoclonal antibody. The protein was visualized as dots distributed throughout the cell (Figures 3 and 4).

Figure 5 shows the analysis of the expression of the recombinant protein L2E6. After transfection of 293F cells with the pcDNA3.3/L2E6 vector, the cells were collected and lysed as previously described. The cell lysate was analyzed on SDS-PAGE 12%, as seen in Figure 5. The theoretical molecular mass of the recombinant L2E6 protein produced would be approximately 3.5 KDa.

The comparison of the running profile of the cell lysate samples from non-transfected and transfected cells revealed the presence of a very low molecular weight protein, below 8 KDa, present only in the transfected samples, suggesting that it is the recombinant protein L2E6. Additionally, Western blotting assays were performed to confirm the expression of the recombinant proteins and the recognition of these proteins by specific commercial monoclonal antibodies anti-L2 and anti-E6, to confirm the identity of the expressed protein. The results obtained revealed that both monoclonal antibodies, anti-L2 and anti-E6, recognized a protein of approximately 3.5 KDa, suggesting confirmation of the identity of the protein of interest (Figure 5).

The photodocumentation of the result obtained from the Western blotting assay used for detection and characterization of the recombinant protein L2E6, where the samples (1) and (5) are aliquots of non-transfected cells lysate, (2) and (6) lysate of transfected cells expressing L2E6, (3) and (7) eluted intermediates obtained from the filtration gel chromatography assay and interacting with the specific monoclonal antibodies anti-L2 (3) and anti-E6 (7), demonstrating the presence of a low molecular weight protein being recognized by both commercial antibodies, (4) final elution sample from the gel filtration process. (MM) Color Burst™ molecular marker.

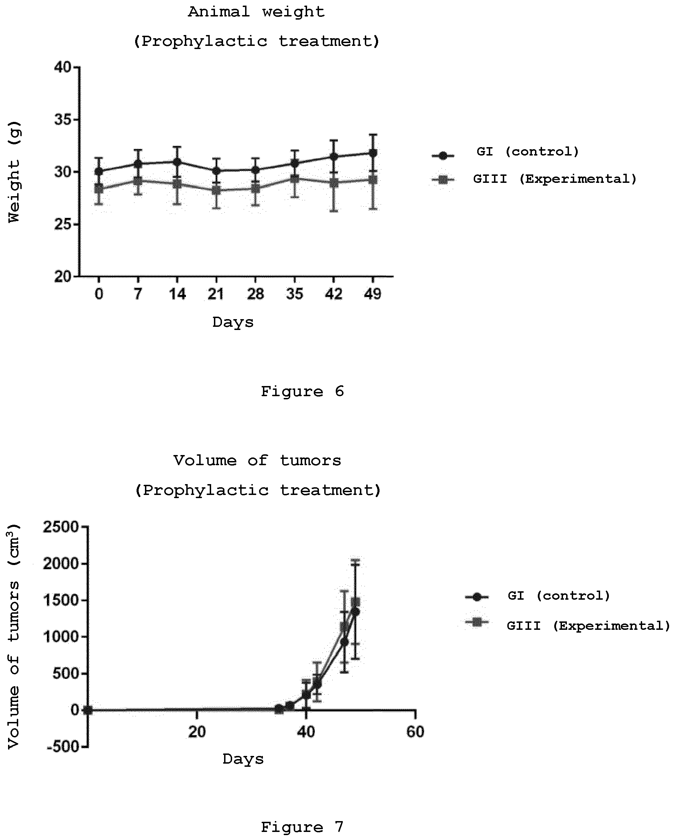

Figure 6 shows the prophylactic evaluation in *in vivo* tests performed in the present work, in experiments to analyze the use of the vector as a DNA vaccine in a murine model. The animals were separated into 4 experimental groups, being evaluated for the possibility of inducing prophylactic and therapeutic protection to the challenge with TC-1 tumor cells.

From the weekly weighing of the animals it was possible to observe that the weight remained constant throughout the experiment. There was a small difference in weight between the animals of the experimental and control group, which received only PBS, since day 0 (zero) experimental, however such difference is not statistically significant, according to Student's t test, suggesting that the vaccine of DNA did not interfere in the animals' weight gain or loss (Figure 6).

Figure 7 shows that one week after the application of the 3 doses of the DNA vaccine proposed in the present study, the animals were challenged with tumor cells TC-1 to assess whether there was an induction of protective immune response and its prophylactic protective capacity, in the tumor development of HPV-positive cells. From the moment the tumors were established and became palpable, they were measured every 2 days and the data analyzed. The tumor volume increased throughout the experiment, demonstrating that there was a tumor implantation with tumor development. There was no statistically significant difference between animals in the same group, just as it was not possible to observe a difference in the tumor volume of animals between experimental and control groups (Figure 7), suggesting that the DNA vaccine used prophylactically did not induce an immune response capable of prevent or control tumor development. However, such a result was expected, since the prophylactic capacity of the developing vaccine would be limited in its ability to induce the production of specific antibodies capable of preventing the infection of cells by HPV.

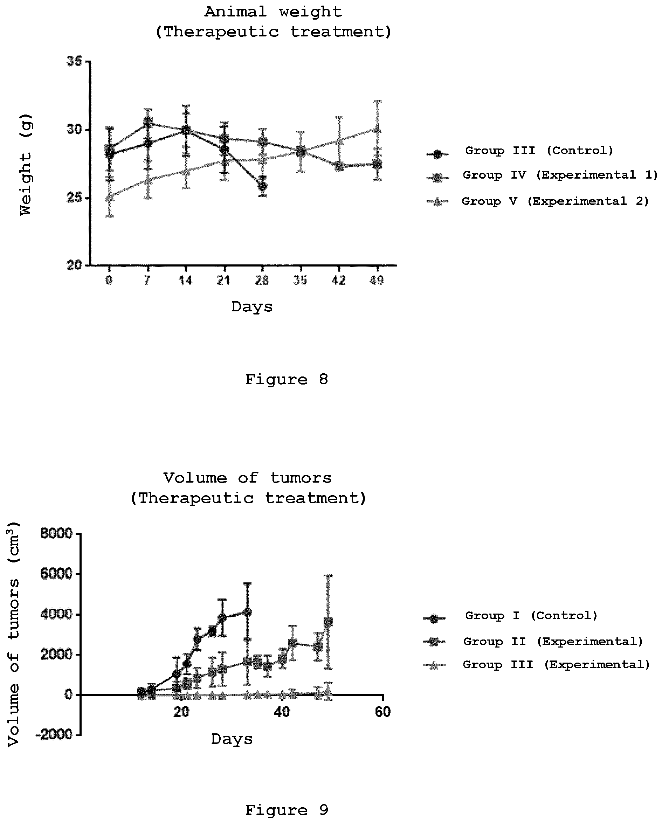

Figure 8 shows the therapeutic evaluation of the DNA vaccine. Throughout the experimental procedure, the animals were weighed weekly to monitor their development and possible changes induced by the application of DNA vaccines. TC-1 cells, when applied subcutaneously, have the ability to establish and form solid and non-invasive tumors, however their growth is fast and capable of inducing the formation of external wounds on the animals' skin. Thus, after application of the tumor cells, the animals were monitored for tumor development, and when they induced formation of exposed wounds or when the largest diameter of the tumor mass reached 200 mm, the animals were euthanized. Thus, all animals in the control group, group II, which received tumor cells and did not receive the DNA vaccine, receiving only the empty vector, were euthanized on the twenty-eighth experimental day. Thus, in the group of animals that received the tumor cells and the treatment with the empty vector, it was possible to observe that the tumor implantation induced the weight loss of the animals since the seventh day after the inoculation of the neoplastic cells, however this weight difference is not statistically significant. Only on the twenty-eighth experimental day it was possible to observe a great decrease in weight in the animals (Groups III and IV, Figure 8), being statistically significant when compared to the previous weighings ($p > 0.0001$). Within the same group there was no significant difference in the animals' weight (Figure 8). The animals that received tumor cells and the pcDNA3.3/L2E6 vaccine did not demonstrate significant weight loss during the entire experiment, including the group that received the vaccines in shorter periods between doses, group V, the growth of the animals showed to be continuous throughout the experiment.

Figure 9 shows that from the tenth day after inoculation of TC-1 cells it was already possible to detect the presence of a palpable tumor mass, but not yet measurable in all animals in group III, which received the vaccine containing the vector empty. However, with the rapid growth of tumors, measurements of tumor diameters were started after 2 days, followed by measurements every 2 days. The data obtained demonstrate rapid tumor growth, with similar tumor volumes among animals in the same group. Regarding animals of group IV, which received 3 doses of pcDNA3.3/L2E6 vaccine at 15-day intervals, 3 animals in the group developed solid tumors from the 12th experimental day on. With the rapid development of the tumors, these 3 animals were euthanized on the twenty-eighth experimental day. The remaining 7 animals in the group did not develop tumors throughout the analyzed period.

Regarding animals of group V, which received only 2 doses of the pcDNA3.3/L2E6 vaccine at 10-day intervals, 8 of them did not develop any tumor associated with the application of TC-1 cells, being maintained throughout the experimental period. Two animals in this group developed a tumor, but with much smaller volumes when compared to animals in the control group, III.

Among groups, the difference in tumor volumes was statistically significant ($p < 0.0001$), immediately after the immunization of the second dose of the DNA vaccine, suggesting that it induced an immune response sufficiently capable of containing tumor growth in animals of group IV. Over time, this difference in tumor volume between groups III and IV became more evident, as can be seen in Figure 9. The tumor volumes of animals in the group that received the DNA vaccine, group IV, remained increased, although only slightly, suggesting stability up to 14 days after the last dose, when the measurements again showed a small increase, but not statistically significant. The animals in group V, on the other hand, those that presented the formation of tumor masses, were growing at a very slow pace, when compared to the other animals that presented tumor development. And the final volume reached by these tumors was much smaller when compared to the other animals.

Table 2 shows the analysis of cytokine production.

From the sera obtained from the animals used in the experiments, cytokine detection and dosage tests were performed in an attempt to trace an immune response profile induced by the DNA vaccine.

A pool of serum samples from days 0, 28 and 49 were separated by groups and with the aid of the BD CBA Mouse Th1/Th2 Cytokine kit. In all tested samples it was possible to detect the presence of the analyzed cytokines, however the results obtained for the IFN, IL-2, IL-4 and IL-5 cytokines, the concentrations found were low and it was not possible to observe differences in the concentrations of the samples collected before and after treatments, as can be seen in Table 2, below (Table 2).

The statistical analysis using the Mann & Whitney non-parametric test revealed that there was no significant difference in the TNF concentrations detected between the control and experimental groups ($p < 0.05$).

Table 2 - Levels of cytokines detected in serum samples.

| Groups | IFN (pg/mL) | | IL-2 (pg/mL) | | IL-4 (pg/mL) | | IL-5 (pg/mL) | |
|---|---|---|---|---|---|---|---|---|
| | Day 0 | Day 49 | Day 0 | Day 49 | Day 0 | Day 49 | Day 0 | Day 49 |
| **Group I (Prophylactic - Control)** | N/D[#] | N/D[#] | N/D[#] | N/D[#] | N/D[#] | N/D[#] | N/D[#] | N/D[#] |
| **Group II** | N/D[#] | N/D[#] | N/D[#] | N/D[#] | N/D[#] | N/D[#] | N/D[#] | N/D[#] |
| **(Prophylactic -Experimental)** | | | | | | | | |

(continued)

| Groups | IFN (pg/mL) | | IL-2 (pg/mL) | | IL-4 (pg/mL) | | IL-5 (pg/mL) | |
|---|---|---|---|---|---|---|---|---|
| | Day 0 | Day 49 | Day 0 | Day 49 | Day 0 | Day 49 | Day 0 | Day 49 |
| Group III (Therapeutic - Control) | 16.53 | 11.02 | 10.65 | 9.10 | 11.61 | 8.00 | 13.77 | 9.11 |
| Group IV (Therapeutic - Experimental 1) | 15.13 | 16.23 | 9.96 | 10.81 | 0.79 | 11.45 | 3.85 | 13.70 |
| Group V (Therapeutic - Experimental 2) | 17.03 | 17.64 | 1.14 | 11.18 | 1.77 | 11.12 | 3.92 | 15.35 |

However, in the TNF (tumor necrosis factor) analyzes of the serum samples, it was possible to observe a significant increase in the concentrations of this cytokine on the last experimental day, 49, as can be seen in the table below (Table 3).

Table 3 - Levels of TNF detected in the serum of the animals studied.

| Groups | TNF dosage (pg/mL) | | |
|---|---|---|---|
| | Day 0 | Day 28 | Day 49 |
| I - Prophylactic evaluation, control group | 7.06 | 4.44 | 5.80 |
| II - Prophylactic evaluation, vaccinated group | 4.80 | 5.02 | 4.80 |
| III - Therapeutic evaluation, control group | 4.44 | 6.02 | --- |
| IV - Therapeutic evaluation, vaccinated group, doses in 15 days | 5.95 | 7.52 | 12.33* |
| V - Therapeutic evaluation, vaccinated group, doses in 10 days | 5.08 | 7.17 | 5.12 |

The same cytokine detection analyzes were also performed with the samples of the splenocyte culture supernatant when incubated with aliquots of the synthetic peptide PEP1 (E6). The levels of IFN, IL-2, IL-4 and IL-5 were not detected, as the concentrations found were all outside the standard concentration curve. However, high levels of TNF were detected in samples from animals in groups IV and V, as can be seen in table 4 (below).

Table 4 - TNF levels detected in the supernatant of the splenocyte culture.

| Groups | TNF dosage (pg/mL) |
|---|---|
| III - Therapeutic evaluation, control group | 42.02 |
| IV - Therapeutic evaluation, experimental group, doses in 15 days | 85.31* |
| V - Therapeutic evaluation, experimental group, doses in 10 days | 97.40* |

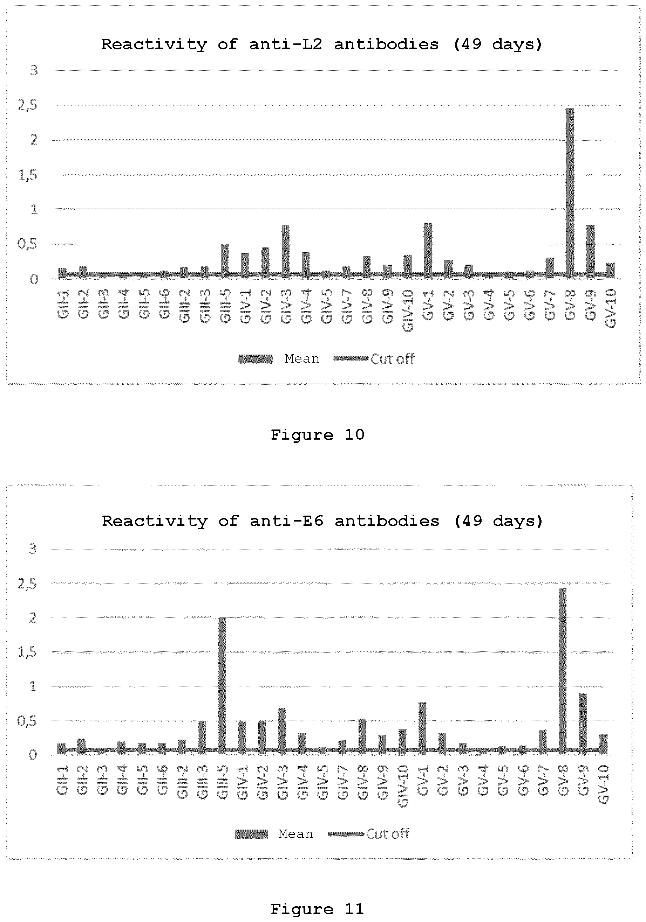

Figures 10 and 11 show the analysis of the production of specific antibodies. Serum samples collected from all animals and groups were analyzed in indirect ELISA assays, to demonstrate whether there was an induction of humoral immune response induced by the pcDNA3.3/L2E6 vaccine with the production of specific anti-L2 or anti-E6 antibodies. The samples were tested in triplicate and their results were analyzed as previously described. The data obtained demonstrated that in the samples of day 0 no anti-L2 or anti-E6 antibodies were detected in any of the animals, as expected. However, in samples on the 28th it was already possible to detect the presence of specific antibodies at high levels, however, the peak of reactivity was found on day 49 and the presence of specific anti-L2 (Figure 10) and anti-E6 antibodies (Figure 11) was then demonstrated. The animals in group IV that developed tumors were the same ones that showed less capacity to produce anti-L2 and anti-E6 antibodies, receiving the numbers 5, 6, 7 and 9. The 2 animals in group V that developed solid tumors also had the lowest levels of production of anti-L2 and anti-E6 antibodies, being 4 and 5. The data of the animals that do not appear in the figures below represent those that had a greater tumor development, not resisting until the 49 experimental days, being euthanized when reaching the previously mentioned parameters.

Thus, although only some embodiments of the present invention have been shown, it will be understood that various omissions, substitutions and changes in the prophylactic and therapeutic DNA vaccine against HPV and cancers associated with the virus can be made by a person skilled in the art, without depart from the spirit and scope of the present invention.

It is expressly provided that all combinations of elements that perform the same function in substantially the same way to achieve the same results are within the scope of the invention. Substitutions of elements from one embodiment described to another are also fully intended and contemplated.

It is also necessary to understand that the drawings are not necessarily to scale, but that they are only of a conceptual nature. The intention is, therefore, to be limited, as indicated by the scope of the attached claims.

## Claims

1. Process for producing prophylactic and therapeutic vaccine against HPV and cancers associated with the virus **characterized by** comprising the following steps:

   a) selection of epitopes and construction of a vaccine vector containing peptides selected from HPV16 L2 and E6 proteins,
   b) amplification of the vaccine vector;
   c) bacterial transformation;
   d) selection of bacterial colonies;
   e) extraction of plasmideal DNA;
   f) DNA sequencing;
   g) cell transfection;
   h) expression and characterization of recombinant proteins;
   i) Protein synthesis.

2. Process, according to claim 1, **characterized in that** in step (b), amplification of the vaccine vector occurs in bacterial cultures of *Escherichia coli* DH5α.

3. Process, according to claim 1, **characterized in that** in step (a), there were used HPV16 L2 and E6 gene sequences which were obtained from the international GenBank(r) database, wherein the access code of the E6 gene used was AAD33252 and the reference for access to L2 was NC_001526.2.

4. Process, according to claim 3, **characterized in that** the peptides selected from the E6 gene are among the amino acid residues 50 to 57 as established in SEQ ID NO: 5.

5. Process, according to claim 1, **characterized in that** the codons have been optimized for greater success in their expression in human cells, using the Codon Optimization tool from IDT - Integrated DNA Technology, and the final sequence obtained was as set out in SEQ ID NO: 4.

6. Process, according to claim 1, **characterized in that** for the L2 gene, the selected peptide was found among amino acid residues 17 to 36 as established in SEQ ID NO: 2, with the optimized sequence resulting in: SEQ ID NO: 3.

7. Process, according to claim 1, **characterized in that** both sequences were linked by 3 "Linker" sequences, resulting in the 96 bp sequence (base pairs), to which the initiator and termination sequences were added, giving rise to the 102 bp sequence as established by SEQ ID NO: 1.

8. Process, according to claim 1, **characterized in that** the final sequence was synthesized giving rise to the synthetic gene called L2E6.

9. Process, according to claim 1, **characterized in that** in step (a), 2 vectors were designed containing the synthetic gene L2E6, being a cloning vector based on the commercial vector pMAT and an expression vector or vaccine vector in pcDNA.

10. Process, according to claim 9, **characterized in that** for the construction of the cloning vector, the synthetic gene L2E6 was inserted in pMA-T, with both ends of the synthetic gene and the vector flanked by endonuclease sequences Sfi I.

**11.** Process, according to claim 10, **characterized in that** the vector called pMA-T/L2E6 has bacterial Col E1 replication origin and ampicillin resistance gene, AmpR.

**12.** Process, according to claim 11, **characterized in that** the complete vector has 2494 bp.

**13.** Process, according to claim 11, **characterized in that** for the construction of the expression vector, the L2E6 insert was inserted in the vector pcDNA3.3 flanked by endonuclease sites Hind III and Nhe I, being named pcDNA3.3/L2E6, in which the constructed plasmid originates from bacterial replication, pUC, Ampicillin resistance gene, Amp (R) and CMV promoter (Cytomegalovirus).

**14.** Process, according to claim 11, **characterized in that** for vector analysis, electrophoresis assays were performed on 0.8% (m/v) agarose gel in 0.5x TAE buffer (Tris 40 mM, 20 mM acetate, 1 mM EDTA) and subjected to an 80V electrophoresis run in the presence of Blue Green Loading Dye.

**15.** Process, according to claim 1, **characterized in that** in step (b) for the amplification of the vectors, the bacterial strains *Escherichia coli* DH5a and TOP10 were used, wherein the bacterial strains were grown in 3 mL of Luria medium-Bertani (LB) (2% tryptone, 1% yeast extract, 1% NaCl) at 37°C, under stirring at 250 rpm for approximately 18 hours, then an aliquot of this culture was streaked into a Petri dish containing LB medium plus 1.5% agar, incubated at 37°C, for approximately 18 hours, where a colony was chosen to sow 3 mL of LB medium and grown under the same conditions described above, after that period, 1 ml of this culture was used to sow 100 mL of LB medium and cultivated at 37°C, under agitation until the optical density of the culture at 600 nm (DO600) reaches 0.4, wherein the cultured bacteria were in the middle of the exponential growth phase.

**16.** Process, according to claim 15, **characterized in that** the competence induction was performed, wherein after reaching the desired cell density, the culture of bacteria was transferred to a conical tube and centrifuged at 5000xg for 10 minutes and incubated in a solution of 0.1 M $CaCl_2$ for 1 hour on ice, then the bacteria were again centrifuged and resuspended in a solution of 0.1 M $CaCl_2$ plus 10% glycerol (w/v), where aliquots of 200 $\mu$L were frozen at -80°C until use.

**17.** Process, according to claim 1, **characterized in that** the bacterial transformation step (c) was carried out from the competent bacteria, where a mixture of 10 $\mu$L of plasmideal DNA, approximately 15 g, and an aliquot of chemo-competent bacteria was incubated for 30 minutes on ice, subjected to a thermal shock for 2 minutes at 42°C and incubated again on ice for 5 minutes, then 350 $\mu$L of S.O.C medium (Tryptone 2%, Yeast extract 0.5%, 10 mM NaCl, 2.5 mM KCl, 10 mM $MgCl_2$, 10 mM $MgSO_4$, 20 mM Glucose), the mixture being incubated for 90 minutes at 37°C, under agitation of 250 rpm, then the transformed bacteria were seeded in plates of LB-agar medium containing or not 100 $\mu$g/ml ampicillin and incubated at 37°C for 18 hours.

**18.** Process, according to claim 17, **characterized in that** colonies grown on LB-agar plates containing ampicillin identify the positive clones that were selected by colony PCR and seeded in tubes containing 3 mL of LB medium, incubated at 37°C, under stirring for approximately 18 hours.

**19.** Process, according to claim 1, **characterized in that** for the selection of bacterial colonies in step (d) after transformation, the colony PCR technique was used, where 2 specific primers synthesized from the L2E6 gene sequence were designed, EAK03 and EAK02, and capable of generating a 144 bp product.

**20.** Process, according to claim 19, **characterized in that** the initiators are as established by SEQ ID NO 6 and SEQ ID NO: 7.

**21.** Process, according to claims 19 and 20, **characterized in that** the primers were resuspended in TE buffer (10 mM Tris / HCl, 1 mM EDTA) at a stock concentration of 100 $\mu$M, in which for PCR, each solution was prepared separately in microtubes containing 25 $\mu$L of PCR-Mix solution (1.5 mM $MgCl_2$, 0.5U Taq, 200 5M dNTPs - LGC Biotecnologia, Cotia, SP, Brazil), 0.5 $\mu$L of each primer at 25 $\mu$M and ultrapure water for a final solution volume of 50 $\mu$L.

**22.** Process, according to claims 19 to 21, **characterized in that,** as template DNA, a sample of each transformed bacterial colony was used, selected and grown in LB medium with ampicillin, wherein the amplification was performed in a thermocycler occurring as the protocol: 95°C for 5 minutes, 25 cycles of 1 minute at 95°C, 30 seconds at 56°C and 1 minute at 72°C, followed by final extension at 72°C for 7 minutes.

**23.** Process, according to claims 19 to 22, **characterized in that** the detection of PCR products occurred through electrophoresis in 0.8% agarose gel in 0.5X TAE buffer. The samples were prepared with the addition of 0.5 $\mu$L of Blue Green Loading Dye I and subjected to a run at 80 A for 30 minutes.

**24.** Process, according to claim 1, **characterized in that** in step (e) of plasmideal DNA extraction, the positive clones selected by the colony PCR were cultured in 5 mL of LB medium, at 37°C, for approximately 18 hours, under agitation, in the presence of the antibiotic of selection, then they were centrifuged at room temperature, for 1 minute at 12000xg to sediment the intact bacteria.

**25.** Process, according to claim 25, **characterized in that** for the purification of plasmideal DNA, the GeneJet Plasmid Miniprep Kit (Thermo Fisher Scientific) was used, where the purified DNA was analyzed in 0.8% agarose gel in 0.5X TAE buffer and submitted to the sequencing technique to confirm the insertion of the gene of interest to the vector, quantified in a spectrophotometer and stored at -20°C.

**26.** Process, according to claim 1, **characterized in that** in step (f) of DNA sequencing, plasmideal DNA samples were sequenced to confirm the correct insertion of the L2E6 gene and its integrity, where the DNA samples purified from the gel were quantified by spectrophotometry, and a volume of 5 $\mu$L of the plasmid was aliquoted and 2.5 $\mu$L of the EAK03 primer was added at a concentration of 5 $\mu$M.

**27.** Process, according to claim 1, **characterized in that** in step (g) the transient transfection of HEK 293T cells was performed by lipofection using the GeneJuice® Transfection Reagents, where the transfection reagent was mixed by vortex to an aliquot of DMEM medium, being incubated for 5 minutes at room temperature; to the mixture, the plasmideal DNA of interest was added, in the appropriate proportion, gently homogenized and incubated for 15 minutes at room temperature, then the mixture Medium/transfection reagent/DNA was added to the cells with complete culture medium, and incubated in oven at 37°C and 5% $CO_2$, where after 8 hours the culture medium was changed, now with a complete medium containing antibiotics and 10% SBF, wherein the incubation continued for approximately 48 hours when the cells were collected and analyzed in immunofluorescence and in SDS-PAGE electrophoresis gel.

**28.** Process, according to claim 27, **characterized in that** in step (g) for 293F cells, the transfection assays were carried out using the 293fectin transfection reagent.

**29.** Process, according to claim 1, **characterized in that** in step (h), HEK 293T cells were grown on 13 mm diameter round coverslips in 12-well culture plates (TPP®), where the easily adherent strains were grown directly on the coverslips in D10 medium, kept in a humid atmosphere, containing 5% $CO_2$, for approximately 18 hours.

**30.** Process, according to claim 29, **characterized in that** in step (h), for the assays using the 293F cells already transfected, said cells were centrifuged in microtubes and the supernatant discarded.

**31.** Process, according to claims 29 and 30, **characterized in that** after transfections, both cell lines were washed 2 times in PBS and fixed in 1% paraformaldehyde solution in PBS, for 1 hour at 4°C, where after successive washes to remove all fixative, said cells were incubated in 5% PBS+BSA solution to block nonspecific bonds and incubated for 1 hour at room temperature, then the cells were washed in PBS for 3 times and then incubated with specific primary anti-HPV16 L2 or anti-HPV16 E6 antibodies diluted in 0.5% BSA and 0.05% Tween 20 in PBS for 1 hour at room temperature, shaking the tube frequently, where after re-washing, the cells were incubated with the respective specific secondary antibodies diluted in 1.5% BSA and 0.01% Tween 20 in PBS for 1 hour at room temperature, with frequent shaking in the tube containing the cells and the solution with the antibodies, wherein the secondary antibodies used were ram AlexaFluor® 488 anti-mouse IgG produced in goat (Molecular Probes®, OR) and AlexaFluor® 633 anti-IgG rabbit produced in goat, where after a last wash in PBS, 293F cells were distributed over coated glass coverslips with 0.5% poly-L-lysine in PBS.

**32.** Process, according to claims 29 to 31, **characterized in that** after each transfection reaction, the cells were lysed in buffer (0.5 M Tris pH 7.4, 0.5 M NaCl, EDTA 0.5 M pH 8.0, 0.2 M EGTA, Triton X-100, protease inhibitor cocktail), centrifuged at 10,000xg for 5 minutes, with the precipitate discarded and the supernatant stored at -20°C until the time of use.

**33.** Process, according to claims 29 to 32, **characterized in that** the positive samples in the dot blot assays proceeded to analyze the expression of recombinant proteins, carried out through Western blotting assays.

34. Process, according to claim 33, **characterized in that** a 16% Tricina-SDS-PAGE gel (Sodium Dodecyl Sulphate Polyacrylamide Gel Electrophoresis) was prepared and submitted to electrophoretic separation (30 V for 300 minutes), under denaturing conditions, containing samples of transfected cell lysates diluted in running buffer, then the gel was stained in Coomassie Blue solution (7% acetic acid, 50% methanol, 0.1% Coomassie Bright Blue R-250) or proteins were transferred to a nitrocellulose membrane for Western blotting assays, after electrophoretic transfer (30 V and 15 mA for 18 hours, or 70 V and 35 mA for 2 hours) in transfer buffer (Tris 0.025 M, Glycine 0.192 M, 20% Methanol) at 4°C, the membrane was stained in a solution containing Ponceau temporary dye (Ponceau S 0.1%, acetic acid 5%) to confirm the transfer and marking of the molecular weight indicators, then the membranes were washed in distilled water to remove temporary staining and incubated in a solution containing 5% skimmed-milk powder diluted in PBST (Tween 20 0.05%, PBS), for 1 hour, at room temperature, under agitation. After that period, the membranes were washed 4 times in PBST and incubated with 1% skimmed-milk powder solutions in PBST containing the primary antibodies anti-HPV16 L2 monoclonal, anti-HPV16 L2 polyclonal produced in rabbit and/or anti-HPV16 E6 for 2 hours at room temperature, under agitation, then the membranes were washed again 4 times in PBST and incubated in a solution containing the respective secondary antibodies anti-mouse IgG or anti-rabbit IgG conjugated with horseradish peroxidase in solution of 1% skimmed-milk powder in PBST, incubated for 2 hours at room temperature, under agitation.

35. Process, according to claim 34, **characterized in that** the samples of cell lysate were analyzed in 16% Tricina-SDS-PAGE electrophoresis gel, the most suitable for the separation of proteins from 1 to 100 KDa.

36. Process, according to claim 35, **characterized in that** the protein samples were quantified and their concentrations adjusted so that each sample contains approximately 15 $\mu$g of proteins, where the protein dosage occurred using the Bradford method (Bio-Rad Laboratories Inc.), where the samples were diluted in sample buffer (Tris-Cl 1 M pH 6.8, SDS 0.8%, bromophenol blue 0.1%, glycerol 40%, b-mercaptoethanol 14, 4 M) and finally submitted to electrophoretic separation (30 V for 300 minutes) in tris-tricine buffer (0.1 M Tris; 0.1 M Tricine; 0.1% SDS, pH 8.5), after separation, the gel was stained with Coomassie blue solution.

37. Nucleic acid sequence **characterized by** comprising the optimized sequence of the L2 gene as established in SEQ ID NO: 3 and the optimized sequence of the E6 gene as established in SEQ ID NO: 4 linked by 3 "Linker" sequences,

38. Nucleic acid sequence, according to claim 37, **characterized in that** it is as established by SEQ ID NO: 1.

39. Hybrid protein **characterized in that** it is encoded by the nucleic acid sequence as defined by claim 37 or 38.

40. Expression vector **characterized by** comprising the nucleic acid sequence as defined by claims 37 and 38.

41. Expression vector, according to claim 40, **characterized in that** for the construction of the expression vector, the L2E6 nucleic acid sequence was inserted into the pcDNA3.3 vector flanked by Hind III and Nhe I endonuclease sites, receiving the name pcDNA3.3/L2E6, in which the constructed plasmid originates from bacterial replication, pUC, Ampicillin resistance gene, Amp (R) and CMV promoter (Cytomegalovirus).

42. Immune composition **characterized by** comprising the hybrid protein as defined by claim 39 or the vector, as defined by claims 40 and 41, and pharmaceutically acceptable vehicles, excipients and carriers.

43. Composition, according to claim 42, **characterized in that** it optionally comprises vaccine adjuvants, particularly cationic lipids and HPV Virus-like particles (VLPs), or without adjuvants.

44. Composition, according to claims 42 to 43, **characterized in that** the composition comprises dosage forms administered intramuscularly and intradermally, preferably administered with syringes and needles, by electroporation, bioinjectors without needles, or by techniques like DNA tattooing, or orally.

45. Use of the hybrid protein, as defined by claim 39, **characterized in that** it is for the preparation of a prophylactic and therapeutic vaccine against HPV and cancers associated with the virus.

46. Use of the expression vector, as defined by claims 40 and 41, **characterized in that** it is for the preparation of a prophylactic and therapeutic vaccine against HPV and cancers associated with the virus.

47. Use, according to claims 45 and 46, **characterized in that** it produces a lasting humoral immune response, capable of stimulating the production of specific anti-L2 and anti-E6 antibodies against HPV infection (prophylactic action), as well as activating the cellular immune response, to fight tumor cells and induced by the expression of cytokines TNF (Tumor Necrosis Factor) or Tumor Necrosis Factor (therapeutic action).

bla promoter

Amp(R)

Ndel(300)

CMV promoter full length

Ncol(426)

SacI(634)

HindIII(743)

HPV16_L2E6

XhoI(879)

3 stops

DraII(1181)

f1 origin

13AC3L3C_HPV16_L2E6_pcDNA3.3_A246

5549 bp

SV40 early promoter

Ncol(1893)

NarI(2196)

PstI(2249)

pUC origin

Neo(R)

Ncol(2628)

Figure 1

Figure 2

Figure 3

Figure 4

Figure 5

Animal weight

(Prophylactic treatment)

Figure 6

Volume of tumors

(Prophylactic treatment)

Figure 7

Figure 8

Figure 9

Figure 10

Figure 11

## INTERNATIONAL SEARCH REPORT

| International application No |
|---|
| PCT/BR2020/050516 |

**A. CLASSIFICATION OF SUBJECT MATTER**
INV. A61K39/12      C07K14/025
ADD.

According to International Patent Classification (IPC) or to both national classification and IPC

**B. FIELDS SEARCHED**

Minimum documentation searched (classification system followed by classification symbols)
A61K  C07K

Documentation searched other than minimum documentation to the extent that such documents are included in the fields searched

Electronic data base consulted during the international search (name of data base and, where practicable, search terms used)

EPO-Internal, WPI Data, BIOSIS, Sequence Search

**C. DOCUMENTS CONSIDERED TO BE RELEVANT**

| Category* | Citation of document, with indication, where appropriate, of the relevant passages | Relevant to claim No. |
|---|---|---|
| A | R. GAMBHIRA ET AL: "Vaccination of Healthy Volunteers with Human Papillomavirus Type 16 L2E7E6 Fusion Protein Induces Serum Antibody that Neutralizes across Papillomavirus Species", CANCER RESEARCH, vol. 66, no. 23, 17 November 2006 (2006-11-17), pages 11120-11124, XP055477620, US ISSN: 0008-5472, DOI: 10.1158/0008-5472.CAN-06-2560 Whole document. ----- | 1-47 |
| A | US 2008/260765 A1 (WU TZYY-CHOOU [US] ET AL) 23 October 2008 (2008-10-23) Whole document, especially the claims ----- | 1-47 |
|  | -/-- |  |

[X] Further documents are listed in the continuation of Box C.       [X] See patent family annex.

* Special categories of cited documents :

"A" document defining the general state of the art which is not considered to be of particular relevance

"E" earlier application or patent but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step when the document is taken alone

"Y" document of particular relevance; the claimed invention cannot be considered to involve an inventive step when the document is combined with one or more other such documents, such combination being obvious to a person skilled in the art

"&" document member of the same patent family

| Date of the actual completion of the international search | Date of mailing of the international search report |
|---|---|
| 11 May 2021 | 21/05/2021 |

| Name and mailing address of the ISA/ | Authorized officer |
|---|---|
| European Patent Office, P.B. 5818 Patentlaan 2 NL - 2280 HV Rijswijk Tel. (+31-70) 340-2040, Fax: (+31-70) 340-3016 | Kools, Patrick |

Form PCT/ISA/210 (second sheet) (April 2005)

## INTERNATIONAL SEARCH REPORT

| International application No |
|---|
| PCT/BR2020/050516 |

| C(Continuation). | DOCUMENTS CONSIDERED TO BE RELEVANT | |
|---|---|---|
| Category* | Citation of document, with indication, where appropriate, of the relevant passages | Relevant to claim No. |
| A | DATABASE Geneseq [Online]<br><br>24 September 2015 (2015-09-24),<br>"HPV16 immunogenic epitope peptide #115.",<br>XP55803742,<br>retrieved from EBI accession no.<br>GSP:BCD09791<br>Database accession no. BCD09791<br>the whole document<br>----- | 1-47 |
| A | DATABASE Geneseq [Online]<br><br>21 April 2016 (2016-04-21),<br>"Human papillomavirus derived peptide, SEQ ID 138.",<br>XP55803751,<br>retrieved from EBI accession no.<br>GSP:BCM56620<br>Database accession no. BCM56620<br>the whole document<br>----- | 1-47 |
| A | WO 2008/026869 A1 (UNIV SUNGKYUNKWAN FOUND [KR]; YANG JOO-SUNG [KR] ET AL.)<br>6 March 2008 (2008-03-06)<br>Whole document, especially the claims<br>----- | 1-47 |

Form PCT/ISA/210 (continuation of second sheet) (April 2005)

## INTERNATIONAL SEARCH REPORT
Information on patent family members

| International application No |
|---|
| PCT/BR2020/050516 |

| Patent document cited in search report | Publication date | Patent family member(s) | Publication date |
|---|---|---|---|
| US 2008260765 A1 | 23-10-2008 | NONE | |
| WO 2008026869 A1 | 06-03-2008 | EP 2059262 A1 | 20-05-2009 |
| | | KR 20080019569 A | 04-03-2008 |
| | | US 2010285058 A1 | 11-11-2010 |
| | | WO 2008026869 A1 | 06-03-2008 |

Form PCT/ISA/210 (patent family annex) (April 2005)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- BR 10037497
- WO 10037497 A
- WO 96126752 A
- WO 2017211886 A
- WO 200238769 A

### Non-patent literature cited in the description

- **GUAN P, HOWELL-JONESR, L] N et al.** Human Papillomavirus types in 115,789 HPV-positive women: a meta-analysis from cervical infection to cancer. *Int J. Cancer,* 2012, vol. 131, 2349-2359
- **WHO.** Strategic Advisory Group of Experts (SAGE) on immunization WHO. World Health Organization, 2017
- **DE SANJOSÉ S ; DIAZ M, CASTELLSAGUÉ et al.** Worldwide prevalence and genotype distribution of cervical human Papillomavirus DNA in women with normal cytology: a meta-analysis. *Lancet Infect Dis,* 2007, vol. 7, 453-459
- **CIANCIARULLO AM.** Prophylaxis against human Papillomavirus. *Rev. Sodebras,* 15 August 2014, vol. 9 (100, http://www.sodebras.com.br/edicoes/N100.pdf
- **CIANCIARULLO AM.** Prophylaxis against human Papillomavirus. *Rev. Sodebras,* 2014, vol. 9 (100), 8-15, http://www.sodebras.com.br/edicoes/N100.pdf
- *INCA,* 2017, http://portalsaude.saude.gov.br.
- **CIANCIARULLO AM.** human Papillomavirus. *Sodebras,* 2014, vol. 9 (100), 8-15, http://www.sodebras.com.br/edicoes/N100.pdf
- *INCA,* 2017, at:http://portalsaude.saude.gov.br
- **HERRERO R. ; GONZALEZ P ; MARKOWITZ LE.** Present status of human Papillomavirus vaccine development and implementation. *Lancet Oncol,* 2015, vol. 16, E206-216
- **WANG D ; LI Z ; XIAO J et al.** Identification of broad-genotype HPV L2 neutralization site for Pan-HPV vaccine development by a cross-neutralizing antibody. *PLOS One,* 2015, vol. 10, E0123944
- **VANDE POL SB ; KLINGELHUTZA J.** *Papillomavirus E6 oncoproteins Virology,* 2013, vol. 445, 115-137
- **KIMS, CHUNGH ; KONGH et al.** Identification of novel immunogenic human Papillomavirus type 16 E7 specific epitopes restricted to HLA-A*b33;03 for cervical cancer immunotherapy. *Yonsei Med J,* 2017, vol. 58, 43-50
- **HANCOCK G ; HELLNER K ; DARRELL L.** Therapeutic HPV vaccines. *Best Practice & Res Clin Obstet Gynecol,* 2017, https://doi.org/10.1016/j.bpobgyn.2017.09.008
- DESENVOLVIMENTO DE UMA VACINA TERAPÊUTICA PARA TUMORES CAUSADOS PELO VÍRUS PAPILOMA HUMANO TIPO 16'' [DEVELOPMENT OF A THERAPEUTIC VACCINE FOR TUMORS CAUSED BY HUMAN PAPILLOMA VIRUS TYPE 16. **BRUNA FELÍCIO MM PORCHIA.** Master's Dissertation. Interunit Postgraduate Program in Biotechnology USP/Instituto Butantan/IPT, 2009